(19) Europäisches Patentamt
European Patent Office
Office européen des brevets

(11) **EP 0 968 181 B1**

(12) **FASCICULE DE BREVET EUROPEEN**

(45) Date de publication et mention de la délivrance du brevet:
**27.04.2005 Bulletin 2005/17**

(21) Numéro de dépôt: **98958294.5**

(22) Date de dépôt: **01.12.1998**

(51) Int Cl.[7]: **C07C 317/04**, C07D 339/06, C07D 311/82, C07C 317/12, C08G 61/02, C08F 232/04, H01M 10/40, H01M 6/16

(86) Numéro de dépôt international:
**PCT/FR1998/002585**

(87) Numéro de publication internationale:
**WO 1999/028292 (10.06.1999 Gazette 1999/23)**

(54) **SELS DE SULFONES PERFLUORES, ET LEURS UTILISATIONS COMME MATERIAUX A CONDUCTION IONIQUE**

SALZE VON PERFLUORIERTEN SULFONEN UND IHRE VERWENDUNG ALS IONISCH LEITFÄHIGE MATERIALIEN

PERFLUORYNATED SULPHONE SALTS, AND THEIR USES AS IONIC CONDUCTION MATERIALS

(84) Etats contractants désignés:
**DE FR GB IT**

(30) Priorité: **01.12.1997 CA 2224046**
**03.02.1998 CA 2228801**

(43) Date de publication de la demande:
**05.01.2000 Bulletin 2000/01**

(73) Titulaires:
- **ACEP INC.**
**Montreal, Quebec H2Z 1A4 (CA)**
- **UNIVERSITE DE MONTREAL**
**Montréal, Québec H3C 3J7 (CA)**
- **CENTRE NATIONAL DE LA RECHERCHE SCIENTIFIQUE (CNRS)**
**75016 Paris (FR)**

(72) Inventeurs:
- **MICHOT, Christophe**
**F-38000 Grenoble (FR)**
- **ARMAND, Michel**
**Montréal, Québec H3T 1N2 (CA)**
- **CHOQUETTE, Yves**
**Sainte-Julie, Québec J3E 1P4 (CA)**
- **GAUTHIER, Michel**
**La Prairie, Québec J5R 1E6 (CA)**

(74) Mandataire: **Sueur, Yvette et al**
**Cabinet SUEUR et L'HELGOUALCH,**
**109, boulevard Haussmann**
**75008 Paris (FR)**

(56) Documents cités:
**EP-A- 0 850 921** **EP-A- 0 850 932**
**US-A- 5 273 840** **US-A- 5 538 812**

- **CHEMICAL ABSTRACTS, vol. 88, no. 18, 1 mai 1978 Columbus, Ohio, US; abstract no. 130067e, P.I. OGOIKO, ET AL.: "Chelate complexes of trifluoromethanesulphonylmalonate with metals" page 612; XP002097853 & UKR. KHIM. ZH., vol. 43, no. 12, 1977, pages 1298-1300,**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 968 181 B1

Printed by Jouve, 75001 PARIS (FR)

## Description

La présente invention a pour objet des compositions ioniques ayant une conductivité ionique élevée, comprenant un sel dans lequel la charge anionique est délocalisée, et leurs utilisations, notamment comme électrolyte.

Il est connu et particulièrement intéressant d'introduire des groupements ioniques dans les molécules ou les polymères organiques possédant des fonctions variées. Les forces coulombiennes correspondent, en effet, aux interactions les plus fortes disponibles au niveau moléculaire, et les groupements ioniques modifient de la manière la plus marquée les molécules auxquelles ils sont attachés. On peut citer les colorants qui sont rendus solubles dans l'eau à l'aide de fonctions sulfonates ou carboxylates.

Cependant les groupements de ce type $-CO_2^-$ $1/mM^{m+}$ ou $-SO_3^-$ $1/mM^{m+}$ ne sont pas dissociés, et ils n'induisent pas de solubilité dans les solvant autres que l'eau ou certains solvants protiques très polaires comme les alcools légers, ce qui restreint considérablement la portée de leur utilisation.

On connaît par ailleurs les sels des composés $1/mM^{m+}$ $^-C(SO_2R_F)_3$ dans lesquels $R_F$ est un groupement perfluoré et $M^{m+}$ un cation à la valence m qui sont solubles et dissociés dans les milieux aprotiques organiques ou les polymères solvatants. Il est cependant considéré que l'existence d'au moins deux groupements perfluoroalkylesulfonyles (en particulier l'existence d'atomes de fluor sur l'atome de carbone en $\alpha$ de chacun des groupements sulfonyles) qui exercent un pouvoir attracteur important sur les électrons de la charge anionique, est une condition nécessaire à l'obtention des propriétés de solubilité et de dissociation.

D'une manière surprenante, les inventeurs ont trouvé que les excellentes propriétés de solubilité et de dissociation des groupements ioniques $^-C(SO_2R_F)_3$ étaient conservées lorsqu'un seul groupement sulfoné possédait des atomes de fluor sur des atomes adjacents à l'atome de soufre, laissant un choix de molécules fonctionnelles extrêmement large. D'une manière tout aussi inattendue, il a été constaté qu'il était possible, pour l'obtention des mêmes propriétés, d'omettre le groupement $-SO_2$ fixé au groupement non perfluoré à condition que le groupe rattaché directement au carbone ait un paramètre de Hammett $\sigma^*$ supérieur à 0,55. A titre de comparaison, le paramètre de Hammett $\sigma^*$ d'un groupement $-SO_2-$ relié à un groupement non perfluoré est de 3,5 et de 4,55 pour un groupement $CF_3SO_2-$.

La présente invention a pour objet une composition ionique comprenant au moins un composé ionique en solution dans un solvant, ledit composé ionique comprenant une partie anionique associée à au moins une partie cationique $M^{m+}$ en nombre suffisant pour assurer la neutralité électronique de l'ensemble. Ladite composition est caractérisée en ce qu'elle a une conductivité supérieure à $10^{-5}$ S.cm$^{-1}$ à une température comprise entre -30°C et 150°C, en ce que $M^{m+}$ est un proton, un hydronium, un hydroxonium, un nitrosonium $NO^+$, un ammonium $-NH_4^+$, ou un cation ayant la valence m choisi parmi les cations métalliques, les cations organiques et les cations organo-métalliques, et en ce que la partie anionique répond à l'une des formules

$$X_F\text{-}SO_x\text{-}C^-(Z)(Z'),\ X_F\text{-}SO_x\text{-}C^-(YR)(Y'R')\ \text{ou}\ X_F\text{-}SO_x\text{-}C^-(Z)(YR)$$

dans lesquelles :

- x est 2 (groupement sulfonyle) ou 1 (groupement sulfinyle) ;
- $X_F$ représente un radical monovalent ou multivalent choisi dans le groupe constitué par les radicaux perhalogénés linéaires, ramifiés ou cycliques du type alkyle, alkylaryle, oxa-alkyle, aza-alkyle, thia-alkyle, alkényle, oxa-alkényle, aza-alkényle, thia-alkényle ou dialkylazo, ou dans le groupe constitué par les radicaux organiques dans lesquels le carbone en $\alpha$ du groupe $SO_x$ porte au moins un atome F, étant entendu qu'un radical $X_F$ multivalent est relié à plus d'un groupe $-SO_xC-$ ;
- Z et Z' représentent indépendamment l'un de l'autre un radical électro-attracteur monovalent ou multivalent, étant entendu qu'un radical Z ou Z' multivalent peut être relié à plusieurs groupes $-C^-SO_x-X_F$ ;
- Y ou Y' représentent un groupe sulfonyle, sulfinyle, carbonyle ou phosphonyle ;
- R est un radical organique monovalent ou multivalent et R' est H ou un radical organique monovalent ou multivalent, R et R' étant différents d'un perfluoro alkyle lorsque x=2 ; étant entendu que chacun des substituants R, R', Z ou Z' peut faire partie d'un polymère, et que chacun des substituants $X_F$, R, R', Z ou Z' peut être relié à un substituant $X_F$, R, R', Z ou Z' porté par le même centre anionique $C^-$ ou par un autre centre anionique.

Dans le composé ionique d'une composition de la présente invention, le cation peut être un cation métallique choisi parmi les cations de métaux alcalins, les cations de métaux alcalino-terreux, les cations de métaux de transition à l'état divalent ou trivalents, et les cations de terres rares. A titre d'exemple, on peut citer $Na^+$, $Li^+$, $K^+$, $Sm^{3+}$, $La^{3+}$, $Ho^{3+}$, $Sc^{3+}$, $Al^{3+}$, $Y^{3+}$, $Yb^{3+}$, $Lu^{3+}$, $Eu^{3+}$.

Le cation peut également être un cation organométallique, notamment un métallocénium. A titre d'exemple, on peut citer les cations dérivés du ferrocène, du titanocène, du zirconocène, d'un indénocénium ou d'un arène métallocénium, les cations des métaux de transition complexés par des ligands de type phosphine possédant éventuellement une chiralité, les cations organométalliques possédant un ou plusieurs groupements alkyles ou aryles fixés d'une manière covalente à un atome ou un groupe d'atomes, tels les cations méthylzinc, phénylmercure, trialkylétain ou trialkylplomb. Le cation organométallique peut faire partie d'une chaîne polymère.

Le cation du sel d'une composition de l'invention peut être un cation organique choisi dans le groupe constitué par les cations $R"_3O^+$ (onium), $NR"_4{}^+$ (ammonium), $R"C(NHR"_2)_2{}^+$ (amidinium), $C(NHR"_2)_3{}^+$ (guanidinium), $C_5R"_6N^+$ (pyridinium), $C_3R"_5N_2{}^+$ (imidazolium), $C_2R"_4N_3{}^+$ (triazolium), $C_3R"_7N_2{}^+$ (imidazolinium), $SR"_3{}^+$ (sulfonium), $PR"_4{}^+$ (phosphonium), $IR"_2{}^+$ (iodonium), $(C_6R"_5)_3C^+$ (carbonium), les radicaux R" représentant de manière indépendante H ou un radical organique non perfluoré. Les radicaux organiques R", identiques ou différents, sont choisis de préférence dans le groupe constitué par :

- le proton, les radicaux alkyles, alkényles, oxa-alkyles, oxa-alkényles, aza-alkyles, aza-alkényles, thia-alkyles, thia-alkényles, sila-alkyles éventuellement hydrolysables, sila-alkényles éventuellement hydrolysables et les radicaux dialkylazo, lesdits radicaux pouvant être linéaires, ramifiés ou cycliques ;
- les radicaux cycliques ou hétérocycliques comprenant éventuellement au moins une chaîne latérale comprenant des hétéroatomes tels que l'azote, l'oxygène ou le soufre ;
- les groupes aryl, arylalkyl, alkylaryl et alkénylaryle, comprenant éventuellement des hétéroatomes dans le noyau aromatique ou dans un substituant ;
- les groupes comprenant plusieurs noyaux aromatiques ou hétérocycliques, condensés ou non, contenant éventuellement au moins un atome d'azote, d'oxygène, de soufre ou de phosphore. Lorsqu'un cation onium porte au moins deux radicaux R" différents de H, ces radicaux peuvent former ensemble un cycle aromatique ou non, englobant éventuellement le centre portant la charge cationique.

Un groupe cationique onium peut être polycationique, par exemple lorsqu'un substituant R" est un substituant divalent relié à deux centres cationiques. A titre d'exemple, on peut citer le cation du composé suivant :

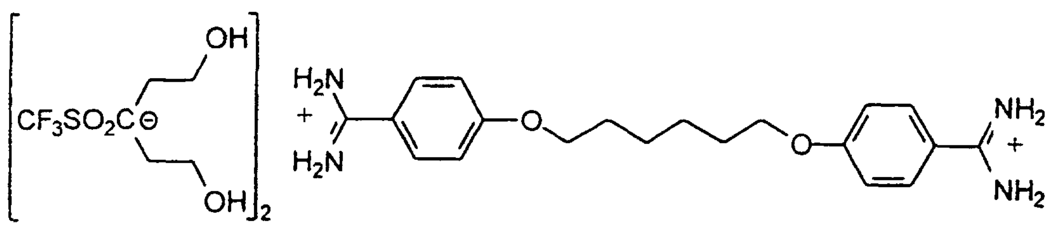

Lorsque la partie cationique du composé ionique est un cation onium, il peut se présenter soit sous la forme d'un groupe cationique indépendant qui n'est lié à la partie anionique que par la liaison ionique entre la charge positive du cation et la charge négative de la partie anionique. Dans ce cas, la partie cationique peut faire partie d'une unité récurrente d'un polymère.

Un cation onium peut également faire partie de l'un des radicaux $R^H$, R ou R'. Dans ce cas, le composé ionique constitue un zwitterion. A titre d'exemple, on peut citer le composé suivant :

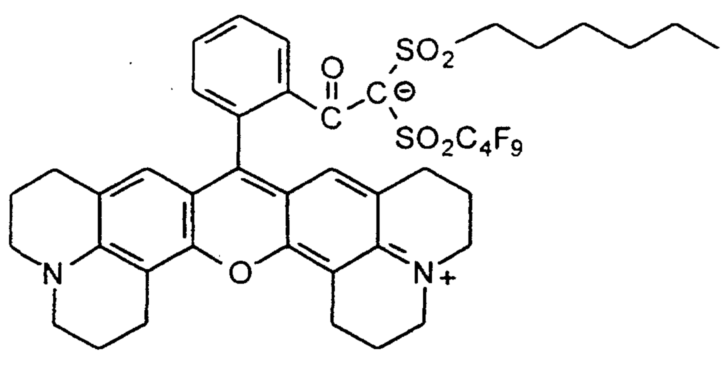

Le cation onium peut être choisi de telle sorte à introduire dans le sel des substituants permettant de conférer

audit sel des propriétés spécifiques. Par exemple, le cation $M^+$ peut être un hétérocycle cationique à caractère aromatique, comportant au moins un atome d'azote quaternisé dans le cycle. A titre d'exemple, on peut citer les ions imidazolium, triazolium, pyridinium et 4-diméthylamino-pyridinium, lesdits cations portant éventuellement un substituant sur les atomes de carbone du cycle. Parmi ces cations, ceux qui donnent un sel dont le point de fusion est inférieur à 150°C sont intéressants, car ils donnent des compositions ioniques qui ont une conduction protonique. Une composition à conduction protonique particulièrement préférée comprend un sel dans lequel le cation est formé par addition d'un proton sur l'azote d'une imidazoline, d'un imidazole ou d'un triazole, ainsi que la base azotée correspondante dans une proportion de 0,5 à 10 en rapport molaire.

Un sel dans lequel le cation est un groupement cationique possédant une liaison -N=N- ou $\sim N{\equiv}N^+$, un groupement sulfonium, un groupement iodonium, ou un cation arène-ferrocénium substitué ou non, éventuellement incorporé dans une trame polymérique, est intéressant dans la mesure où il est activable par une source d'énergie actinique de longueur d'onde appropriée. Comme exemples particuliers de tels sels, on peut citer les sels de diaryliodonium, de dialkylaryliodonium, de triarylsulfonium, de trialkylaryle sulfonium, et de phénacyl-dialkyl sulfonium substitué ou non. Les cations précités peuvent faire partie d'une chaîne polymère.

Le cation M d'un sel peut incorporer un groupement 2,2'[Azobis(2-2'-imidazolinio-2-yl)propane]$^{2+}$ ou 2,2'-Azobis-(2-amidiniopropane)$^{2+}$. Le sel est alors capable de libérer, sous l'action de la chaleur ou d'un rayonnement ionisant, des radicaux qui permettent d'initier des réactions de polymérisation, de réticulation ou, d'une manière générale, des réactions chimiques mettant en jeu des radicaux libres. De plus, ce sel est aisément soluble dans les solvants organiques polymères et monomères même de faible polarité, contrairement aux dérivés des anions de type $Cl^-$ habituellement associés aux cations précités. Ils présentent par ailleurs une pression de vapeur négligeable contrairement aux autres amorceurs radicalaires de type peroxyde ou azo, ce qui est un avantage considérable pour la mise en oeuvre de polymères en films minces, la volatilité de l'amorceur ayant pour conséquence une mauvaise polymérisation ou réticulation de la surface du film.

Dans un mode de réalisation de l'invention, $X_F$ est un radical alkyle perhalogéné ayant de préférence de 1 à 12 atomes de carbone, ou un radical alkylaryle perhalogéné ayant de préférence de 6 à 9 atomes de carbone. Un hétéroatome tel que O, N ou S peut être présent entre deux atomes de carbone ou à l'extrémité du radical. A titre d'exemples, on peut citer les composés suivants :

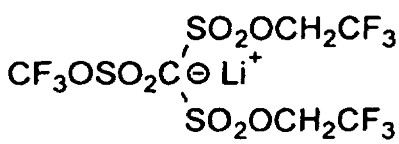

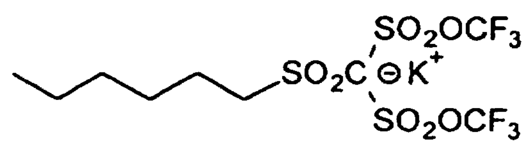

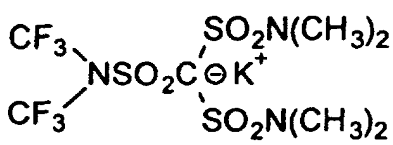

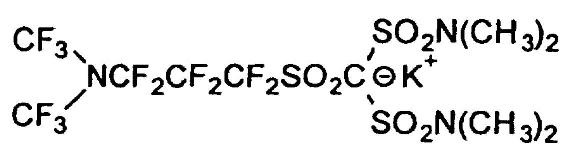

Un substituant $X_F$ peut également être un radical choisi dans le groupe constitué par $R^HCF_2$-, $R^HCF_2CF_2$-, $R^HCF_2CF(CF_3)$- et $CF_3C(R^H)F$-, dans lesquels $R^H$ représente un radical organique non perhalogéné.

Un substituant R ou, R' est choisi parmi ou $R^H$ peut être choisi parmi les radicaux alkyle, alkényle, oxa-alkyle, oxa-alkényle, aza-alkyle, aza-alkényle, thia-alkyle, thia-alkényle et dialkylazo ayant de 1 à 24 atomes de carbone, ou parmi les radicaux aryle, arylalkyle, alkylaryle ou alkénylaryle ayant de 5 à 24 atomes de carbones, lesdits radicaux pouvant éventuellement être totalement ou partiellement fluorés, R et R' étant différents d'un perfluoroalkyle lorsque $X_FSO_x$ est un perfluoroalkylsufonyle. Quelques exemples sont donnés ci-après à titre d'illustration.

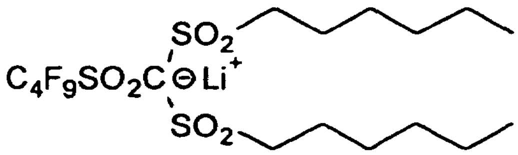

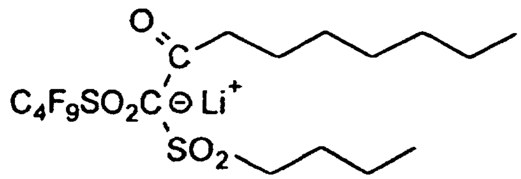

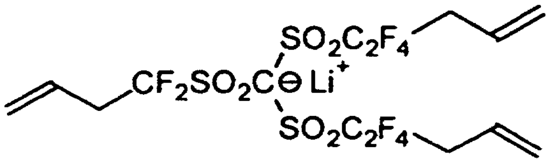

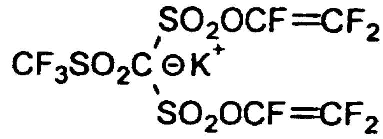

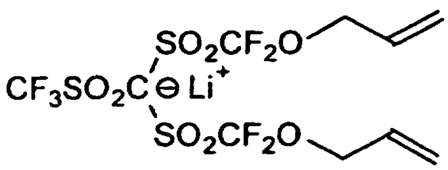

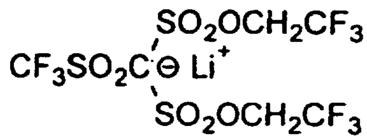

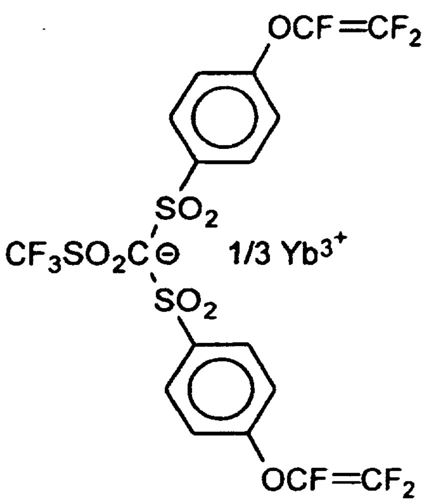

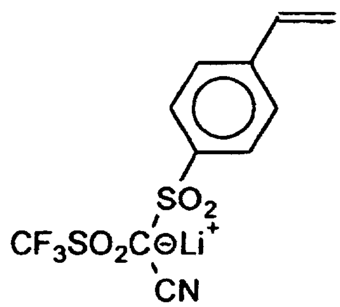

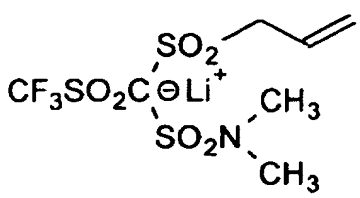

Un substituant R, R' ou $R^H$ peut être choisi parmi les radicaux alkyles ou alkényles ayant de 1 à 12 atomes de carbone et comprenant éventuellement au moins un hétéroatome O, N ou S dans la chaîne principale ou dans une chaîne latérale, et/ou portant éventuellement un groupe hydroxy, un groupe carbonyle, un groupe amine, un groupe alcoxysilane ou un groupe carboxyle. Lorsque deux substituants forment ensemble un radical divalent, ce radical

divalent peut porter un hétéroatome dans une chaîne latérale

Deux radicaux R et R' peuvent former ensemble un biradical relié à chacune de ses extrémités à un groupe anionique $-SO_2-C^{-}SO_2X_F$. On obtient ainsi un composé polyionique, tel que par exemple :

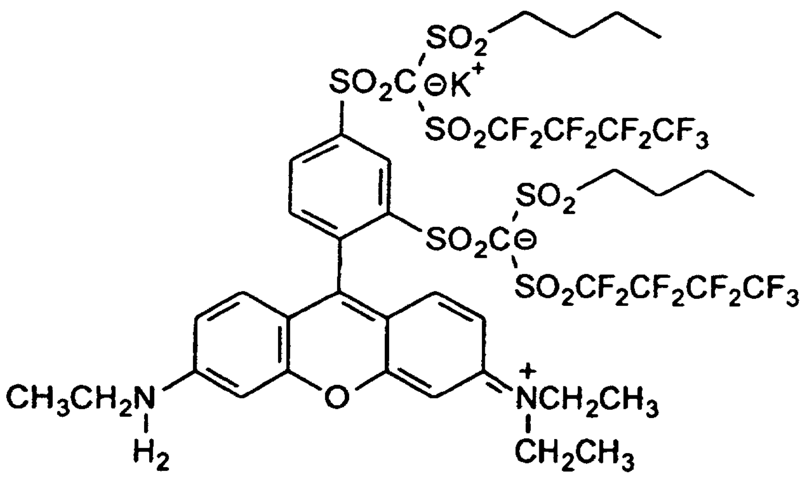

Un substituant R, R' ou $R^H$ peut être un radical polymère, par exemple un radical oligo(oxyalkylène). Le sel de la composition de l'invention se présente alors sous la forme d'un polymère portant un groupe ionique $[(R'Y')(X_FSO_x)CY-]^-$, $M^+$, lorsque R est un radical polymère, ou sous la forme d'un polymère portant un groupe $[(RY)(R'Y')C-SO_x-]^-$ lorsque $R^H$ est un radical polymère.

Un substituant R ou R' peut être une unité récurrente d'un polymère. Le sel de la composition de l'invention se présente alors sous la forme d'un polymère dans lequel une partie au moins des unités récurrentes portent un groupe latéral sur lequel est fixé un groupe ionique $(X_FSO_x)CY-^-$, $M^+$. Comme exemple, on peut citer un poly(oxyalkylène), une poly aniline ou un polyfurane-vinylène dans lequel au moins certaines unités oxyalkylène portent un substituant $[(R'Y')(X_FSO_x)CY-]^-$, $M^+$ ou $[(RY)(R'Y')C-SO_x-]^-$. On peut également citer un polystyrène dans lequel au moins certaines unités styrène portent un substituant $[(R'Y')(X_FSO_x)CY-]^-$, $M^+$ par exemple un substituant [styrényl-(R'Y')$(X_FSO_x)CY-]^-$, $M^+$.

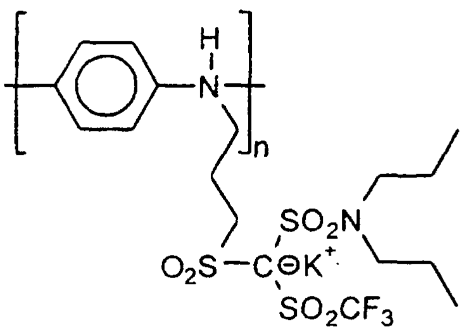

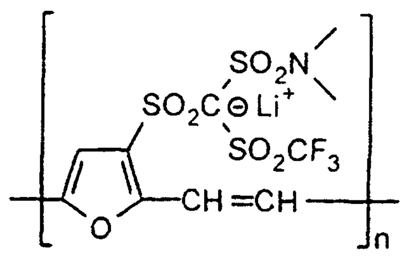

Une catégorie particulière de sels utilisables dans une composition selon l'invention comprend les composés dans lesquels un substituant R, R' ou $R^H$ possède au moins un groupement ionophore anionique et/ou au moins un groupement ionophore cationique. Le groupement anionique peut par exemple être une fonction carboxylate ($-CO_2^-$), une fonction sulfonate ($-SO_3^-$), une fonction sulfonimide ($-SO_2NSO_2^-$) ou une fonction sulfonamide ($-SO_2N=$). Le groupement ionophore cationique peut par exemple être un groupement iodonium, sulfonium, oxonium, ammonium, amidinium, guanidinium, pyridinium, imidazolium, imidazolinium, triazolium, phosphonium ou carbonium. Le groupement ionophore cationique peut jouer totalement ou partiellement le rôle du cation M.

Lorsqu'un substituant R, R' ou $R^H$ comporte au moins une insaturation éthylénique et/ou un groupe condensable et/ou un groupe dissociable par voie thermique, par voie photochimique ou par dissociation ionique, les composés de l'invention sont des composés réactifs qui peuvent être soumis à des polymérisations, des réticulations ou des condensations, éventuellement avec d'autres monomères. Ils peuvent également être utilisés pour fixer des groupes

ionophores sur les polymères portant la fonction réactive appropriée.

Un substituant R, R' ou $R^H$ peut comporter un groupe mésomorphe ou un groupe chromophore ou un polymère conducteur électronique autodopé ou un alcoxysilane hydrolysable.

Un substituant R, R' ou $R^H$ peut comporter un groupement susceptible de piéger les radicaux libres, par exemple un phénol encombré ou une quinone.

Un substituant R, R' ou $R^H$ peut aussi comporter un dipôle dissociant, par exemple une fonction amide, une fonction sulfonamide ou une fonction nitrile.

Un substituant R, R' ou $R^H$ peut aussi comporter un couple rédox, par exemple un groupe disulfure, un groupe thioamide, un groupe ferrocène, un groupe phénothiazine, un groupe bis(dialkylaminoaryle), un groupe nitroxyde ou un groupe imide aromatique.

Un substituant R, R' ou $R^H$ peut aussi comporter un ligand complexant.

Un substituant R, R', $R^H$, RY ou R'Y peut être un groupe optiquement actif (par exemple un acide aminé), ou un polypeptide optiquement ou biologiquement actif.

Lorsque l'anion d'un composé de la présente invention répond à la formule $X_F$-$SO_x$-$C^-$-(Z)(Z') ou $X_F$-$SO_x$-$C^-$-(Z)(YR), Z ou Z' est choisi dans le groupe constitué par -$NO_2$, -SCN, -$N_3$, -$CF_3$, $R'_FCH_2$- ($R'_F$ étant un radical perfluoré), $CF_2$=CFO-, $CF_2$=CF-S-, $CF_2$=CF-, -$C_2F_4H$ les radicaux fluoroalkyloxy ou perfluoroalkyloxy, les radicaux fluoroalkylthioxy et les radicaux perfluoroalkylthioxy. Z ou Z' peut également être un radical comprenant un ou plusieurs noyaux aromatiques contenant éventuellement au moins un atome d'azote, d'oxygène, de soufre ou de phosphore, lesdits noyaux pouvant être éventuellement des noyaux condensés et/ou lesdits noyaux pouvant éventuellement porter au moins un substituant choisi dans le groupe constitué par les halogènes, -$OC_nF_{2n+1}$, -$OC_2F_4H$, -$SC_nF_{2n+1}$, -$SC_2F_4H$, -O-CF=$CF_2$, -SCF=$CF_2$, -CN, -$NO_2$, -SCN, -$N_3$, -$CF_3$, $CF_3CH_2$-, les radicaux aza, thia et oxa, les radicaux perfluoroalkyles, les radicaux fluoroalkyloxy, les radicaux fluoroalkylthioxy, les radicaux alkyles, alkényles, oxa-alkyles, oxa-alkényles, aza-alkyles, aza-alkényles, thia-alkyles, thia-alkényles, les radicaux polymères, et les radicaux possédant au moins un groupement ionophore cationique et/ou au moins un groupement ionophore anionique ;

étant entendu qu'un substituant Z ou Z' peut être un radical monovalent, une partie d'un radical multivalent relié à plusieurs groupements $X_F$-$SO_x$-C<$^-$, ou un segment d'un polymère, et que deux substituants Z et Z' peuvent former ensemble un groupement multivalent comprenant un noyau ou plusieurs noyaux aromatiques éventuellement condensés, incluant ou non le carbone portant la charge anionique.

Un substituant Z peut aussi représenter une unité récurrente d'un polymère. Dans ce cas, la composition ionique de l'invention contient un polymère dans lequel au moins certaines unités récurrentes portent un groupe ionique >$CSO_2X_F$. Le substituant Z d'un groupement anionique et le substituant Z' d'un groupement anionique voisin peuvent également former un biradical. Dans ce cas, la composition ionique de l'invention contient un polymère dans lequel le centre anionique $C^-$ fait partie de la trame du polymère, le groupement $X_FSO_2$ se trouvant en substituant latéral.

Le solvant de la composition ionique de l'invention peut être un solvant liquide aprotique, un polymère polaire ou un de leurs mélanges.

Le solvant liquide aprotique est choisi par exemple parmi les éthers linéaires et les éthers cycliques, les esters, les nitriles, les dérivés nitrés, les amides, les sulfones, les sulfolanes, les alkylsulfamides et les hydrocarbures partiellement halogénés. Les solvants particulièrement préférés sont le diéthyléther, le diméthoxyéthane, le glyme, le tétrahydrofurane, le dioxane, le diméthyltétrahydrofurane, le formiate de méthyle ou d'éthyle, le carbonate de propylène ou d'éthylène, les carbonates d'alkyles (notamment le carbonate de diméthyle, le carbonate de diéthyle et le carbonate de méthylpropyle), la butyrolactone, l'acétonitrile, le benzonitrile, le nitrométhane, le nitrobenzène, la diméthylformamide, la diéthylformamide, les N-alkylpyrrolidones (notamment la N-méthyl-pyrrolidone), la diméthylsulfone, la tétraméthylène sulfone et les tétraalkylsulfonamides ayant de 5 à 10 atomes de carbone.

Une composition ionique de l'invention peut contenir un solvant polymère polaire, choisi parmi les polymères solvatants, réticulés ou non, portant ou non des groupes ioniques greffés. Un polymère solvatant est un polymère qui comporte des unités solvatantes contenant au moins un hétéroatome choisi parmi le soufre, l'oxygène, l'azote et le fluor. A titre d'exemple de polymères solvatants, on peut citer les polyéthers de structure linéaire, peigne ou à blocs, formant ou non un réseau, à base de poly(oxyde d'éthylène), ou les copolymères contenant le motif oxyde d'éthylène ou oxyde de propylène ou allylglycidyléther, les polyphosphazènes, les réseaux réticulés à base de polyéthylène glycol réticulé par des isocyanates ou les réseaux obtenus par polycondensation et portant des groupements qui permettent l'incorporation de groupements réticulables. On peut également citer les copolymères à blocs dans lesquels certains blocs portent des fonctions qui ont des propriétés rédox. Bien entendu, la liste ci-dessus n'est pas limitative, et tous les polymères présentant des propriétés solvatantes peuvent être utilisés.

Une composition ionique de la présente invention peut comprendre simultanément un solvant liquide aprotique choisi parmi les solvants liquides aprotiques cités ci-dessus et un solvant polymère polaire comprenant des unités contenant au moins un hétéroatome choisi parmi le soufre, l'azote, l'oxygène et le fluor. Elle peut comprendre de 2 à 98% de solvant liquide. A titre d'exemple d'un tel polymère polaire, on peut citer les polymères qui contiennent principalement de unités dérivées de l'acrylonitrile, du fluorure de vinylidène, de la N-vinylpyrrolidone ou du méthacrylate

de méthyle. La proportion de liquide aprotique dans le solvant peut varier de 2% (correspondant à un solvant plastifié) à 98% (correspondant à un solvant gélifié).

Une composition ionique de la présente invention peut contenir en outre un sel utilisé classiquement dans l'art antérieur pour l'élaboration d'un matériau à conduction ionique. Parmi les sels qui peuvent être ajoutés à une composition ionique selon l'invention, on peut citer les perfluoroalcanesulfonates, les bis(perfluoroalkylsulfonyl) imidures, les bis(perfluoroalkylsulfonyl) méthanes et les tris(perfluoroalkylsulfonyl)méthanes.

Bien entendu, une composition ionique de l'invention peut contenir en outre les additifs utilisés de manière classique dans les matériaux à conduction ionique, et notamment des charges minérales ou organiques sous forme de poudre ou de fibres.

D'une manière générale, un composé $Q_1C(M)(Q_2)(Q_3)$ dans lequel $Q_1$, $Q_2$ et $Q_3$ représentent RY, R'Y', Z, Z' ou $X_FSO_x$ peut être préparé par action d'un composé possédant un groupe partant L sur un précurseur du composé final, selon le schéma réactionnel suivant :

$$Q_1L + EC(M)(Q_2)(Q_3) \Rightarrow LE + Q_1C(M)(Q_2)(Q_3)$$

L représente un groupe partant tel que F, Cl, Br, imidazolyle, triazolyle, $RSO_3$-. E représente un groupe électrophile tel que M. Par exemple, E peut être H, Li, Na, K, un ammonium substitué ou non, MgL, $Si(R^7)_3$ ou $Sn(R^8)_3$, $R^7$ et $R^8$ représentant un radical alkyloxy.

Un composé dans lequel l'anion est $X_F\text{-}SO_x\text{-}C^-(Z)(Z')$ peut être préparé par action d'un composé possédant un groupe partant L sur un précurseur du composé final, suivant le schéma réactionnel suivant :

$$X_FSO_XL + E(M)C(Z)(Z') \Rightarrow X_FSO_XC(Z)(Z')(M) + L\ E$$

Les composés $EC(M)(Q^2)(Q^3)$ peuvent être préparés par un méthode similaire suivant le schéma réactionnel

$$Q^2L + E_2MCQ^3 \Rightarrow EC(M)(Q^2)(Q^3) + LE$$

dans lequel $Q^2$, $Q^3$, E, M et L ont la signification indiquée ci-dessus.

Dans un variante, lorsque Q représente $X_FSO_X$-, la réaction peut faire intervenir le soufre à l'état d'oxydation +2 ou +4 selon le schéma réactionnel suivant :

$$X_FSO_XE + E\ (L)MCQ^3 \Rightarrow X_FSO_XC(M)(Q^2)(Q^3) + LE$$

Une composition ionique de la présente invention du type polymère peut être préparée par dissolution dans un solvant commun tel l'acétonitrile, d'un polymère solvatant et d'un composé ionique portant un groupement $>^-CSO_xX_F$, puis évaporation du solvant. On peut également mettre en oeuvre un mode opératoire consistant à dissoudre un composé ionique dans un solvant volatil, puis à mettre en solution cette composition ionique et un polymère solvatant dans un solvant commun et à évaporer le solvant volatil, la composition ionique étant choisie de manière à donner un rapport composé ionique/unité de répétition prédéterminé. Une composition ionique de la présente invention du type gel peut être obtenue soit en incorporant un solvant liquide à une composition polymère, soit en dissolvant le composé ionique dans un solvant dans lequel le polymère peut également être dissous à chaud.

Les sels des compositions ioniques de la présente invention comprennent au moins un groupement ionophore sur lequel sont fixés des substituants qui peuvent être très variés. Compte tenu du grand choix possible pour les substituants, les sels permettent d'induire des propriétés de conduction ionique dans la plupart des milieux organiques, liquides ou polymères possédant une polarité, même faible. Les applications sont importantes dans le domaine de l'électrochimie, en particulier du stockage de l'énergie dans des générateurs primaires ou secondaires, dans les supercapacités, dans les piles à combustibles et dans les diodes électroluminescentes. Lorsque la composition selon l'invention contient un sel polymère (introduit directement dans la composition ou obtenu in situ par polymérisation d'un sel monomère), elle présente les propriétés énumérées ci-dessus avec l'avantage d'avoir une charge anionique immobile.

Une composition ionique selon l'invention peut être utilisée comme électrolyte d'un dispositif électrochimique.

Dans ce cas, le sel est choisi de préférence parmi les composés dont le cation est l'ammonium, ou un cation dérivé d'un métal, en particulier le lithium ou le potassium, le zinc, le calcium, les métaux des terres rares, ou un cation organique, tel qu'un ammonium substitué, un imidazolium, un triazolium, un pyridinium, un 4-diméthylamino-pyridinium,

lesdits cations portant éventuellement un substituant sur les atomes de carbone du cycle. L'électrolyte ainsi obtenu présente une conductivité et une solubilité dans les solvants élevées, du fait des interactions faibles entre la charge positive et la charge négative. Son domaine de stabilité électrochimique est étendu, et il est stable dans des milieux aussi bien réducteurs qu'oxydants. Parmi les sels utilisables dans une composition ionique de l'invention, l'on peut citer ceux qui ont un cation organique et un point de fusion inférieur à 150°C, en particulier les sels d'imidazolium, de triazolium, de pyridinium, de 4-diméthylamino-pyridinium. A titre d'exemple, on peut citer les sels suivants :

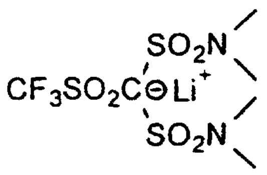
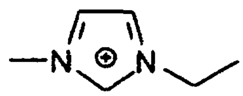
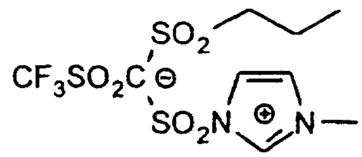

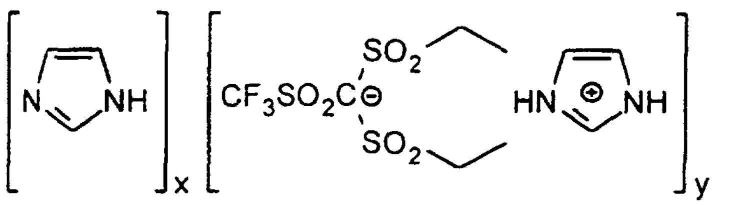

O < x, y < 1
x + y = 1

Ces sels présentent une conductivité élevée intrinsèque lorsqu'ils sont en phase fondue. Ils peuvent donc être utilisés avec une quantité minime, voire nulle de solvant pour former une composition à conduction ionique.

Une composition de l'invention dans laquelle l'anion du sel contient un substituant $X_F$ alkyle perhalogéné ayant de 1 à 12 atomes de carbone, ou un substituant alkylaryle perhalogéné ayant de 6 à 9 atomes de carbone est particulièrement intéressante comme électrolyte dans la mesure où les faibles interactions entre les atomes de fluor de la chaîne entraînent des solubilités et une conductivité élevées, même dans le cas où les autres substituants du sel contiennent des groupements ayant tendance à donner de fortes interactions, par exemple les radicaux aromatiques conjugués ou les zwitterions.

Un composé de l'invention dans lequel $X_F$ est choisi parmi les radicaux $R^HCF_2$-, $R^HCF_2CF_2$-, $R^HCF_2CF(CF_3)$- ou $CF_3C(R^H)F$- permet d'adapter de manière très précise les propriétés du matériau à conduction ionique en choisissant de manière approprié le substituant $R^H$. En particulier, il permet de bénéficier, avec un nombre réduit d'atomes de fluor, des propriétés de dissociation et de solubilité propres aux charges anioniques des systèmes perfluorés. Ces groupements sont aisément accessibles à partir de produits industriels comme le tétrafluoroéthylène ou le tétrafluoropropylène. La quantité réduite de fluor rend ces composé moins sensibles à la réduction par les métaux très électropositifs comme l'aluminium, le magnésium ou surtout le lithium.

Une composition ionique de l'invention peut être utilisée comme électrolyte dans un générateur électrochimique. La présente invention a ainsi pour autre objet un générateur électrochimique comprenant une électrode négative et une électrode positive séparées par un électrolyte, caractérisé en ce que l'électrolyte est un composition ionique tel que définie ci-dessus. Selon un mode de réalisation particulier, un tel générateur comprend une électrode négative constituée par du lithium métallique, ou par l'un de ses alliages, éventuellement sous forme de dispersion nanométrique dans de l'oxyde de lithium, ou par un nitrure double de lithium et d'un métal de transition, ou par un oxyde à bas potentiel ayant pour formule générale $Li_{1+y+x/3}Ti_{2-x/3}O_4$ ($0 \leq x \leq 1$, $0 \leq y \leq 1$), ou par le carbone et les produits carbonés issus de la pyrolyse de matières organiques. Selon un autre mode de réalisation, le générateur comprend une électrode positive choisie parmi les oxydes de vanadium $VO_x$ ($2 \leq x \leq 2{,}5$), $LiV_3O_8$, $Li_yNi_{1-x}Co_xO_2$, ($0 \leq x \leq 1$ ; $0 \leq y \leq 1$), les spinelles de manganèse $Li_yMn_{1-x}M_xO_2$ (M = Cr, Al, V, Ni, $0 \leq x \leq 0, 5$ ; $0 \leq y \leq 2$), les polydisulfures organiques, FeS, $FeS_2$, le sulfate de fer $Fe_2(SO_4)_3$, les phosphates et phosphosilicates de fer et de lithium de structure olivine, ou leurs produits de substitution du fer par le manganèse, utilisés seuls ou en mélanges. Le collecteur de l'électrode positive est de préférence en aluminium.

Comme électrolyte d'un générateur électrochimique, on utilisera de préférence une composition ionique contenant un sel de métal alcalin. On préfère tout particulièrement les sels de lithium, éventuellement en mélange avec un sel de potassium.

Les sels dans lesquels les substituants R et R' représentent un alkyle, un aryle, un alkylaryle, un aralkyle ayant de préférence de 6 à 20 atomes de carbone, constituent de bons candidats pour les compositions ioniques

destinées à être utilisées comme électrolyte d'un générateur électrochimique. Toutefois, des propriétés particulières peuvent être obtenues en choisissant d'autres substituants. Lorsque le sel a au moins un substituant R, R' ou $X_F$ contenant un groupe mésomorphe ou une insaturation éthylénique et/ou un groupe condensable et/ou un groupe dissociable par voie thermique, par voie photochimique ou par dissociation ionique, la composition ionique forme aisément des polymères ou copolymères qui sont des polyélectrolytes, soit intrinsèques quand le polymère porte des groupements solvatants, soit par addition d'un solvant polaire de type liquide ou polymère, ou par mélange avec un tel solvant. Ces compositions du type polyélectrolyte ont une conductivité uniquement due au cations.

Lorsque le sel de la composition ionique utilisée comme électrolyte porte au moins un substituant R, R' ou $R^H$ qui est un polymère conducteur électronique autodopé, la stabilité de l'électrolyte est améliorée par rapport aux agents extérieurs et la conductivité est stable dans le temps même à des températures élevées. En contact avec les métaux, une telle composition donne des résistances d'interface très faibles. Son utilisation comme électrolyte permet de protéger de la corrosion, en particulier les métaux ferreux ou l'aluminium, en particulier lorsque l'aluminium est utilisé comme collecteur de la cathode du générateur. A titre d'exemple, on peut citer :

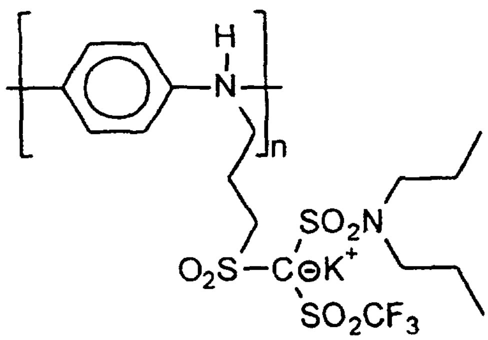

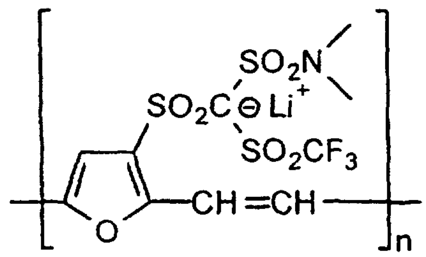

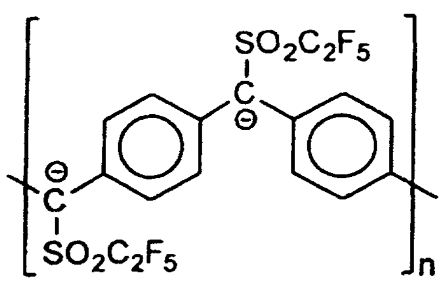

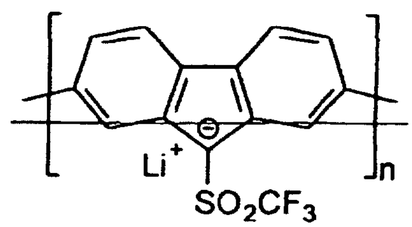

Lorsque le sel de la composition ionique utilisée comme électrolyte contient un substituant R, R' ou $R^H$ qui contient un couple rédox (par exemple un disulfure, une thioamide, un ferrocène, une phénothiazine, un groupe bis (dialkylaminoaryle), un nitroxyde, un imide aromatique), l'électrolyte a des propriétés de navette rédox utiles comme élément de protection et d'égalisation de charge du générateur électrochimique.

L'utilisation d'un sel portant au moins un substituant R, R' ou $R^H$ qui représente un alkyle, un aryle, un alkylaryle ou un arylalkyle, permet d'induire dans l'électrolyte du générateur des propriétés de type mésogène. Les groupes alkyle ayant de 6 à 20 atomes de carbones et les groupes aryle-alkyle, en particulier ceux contenant l'entité biphényle qui forment des phases de type cristal liquide, sont particulièrement préférés. Les propriétés de conduction des phases de type cristal liquide, nématique, cholestérique ou discotique ainsi constituées sont pour réduire la mobilité des anions de l'électrolyte, en particulier dans les électrolytes polymères, sans affecter la mobilité des cations. Cette particularité est importante pour les générateurs électrochimiques mettant en jeu les cations lithium.

Lorsqu'une composition selon l'invention est destinée à être utilisée comme électrolyte d'un générateur au lithium, le solvant est choisi avantageusement parmi les solvants liquides aprotiques polaires utilisés classiquement. Il peut également être choisi parmi les solvants polymères polaires solvatants, réticulés ou non, portant ou non des groupes ioniques greffés. On peut également utiliser comme solvant un gel obtenu par mélange d'un solvant liquide et d'un solvant polymère. Un poly(oxyde d'éthylène) et un copolymère d'oxyde d'éthylène ou d'oxyde de propylène et d'un monomère réticulable tel que l'allylglycidyléther et le méthylglycidyléther sont particulièrement intéressants.

Une composition ionique de l'invention peut également être utilisée pour l'élaboration de la cathode d'un générateur au lithium. Le matériau de la cathode comprend alors une composition ionique de l'invention, dans laquelle le sel est un polymère conducteur électronique. A titre d'exemple d'un tel sel, on peut citer :

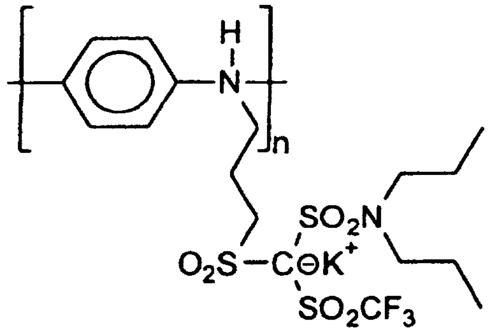

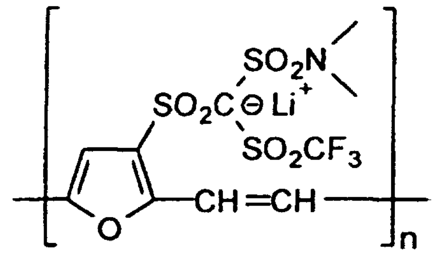

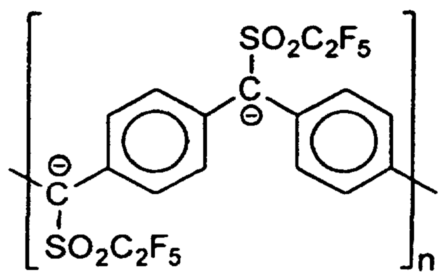

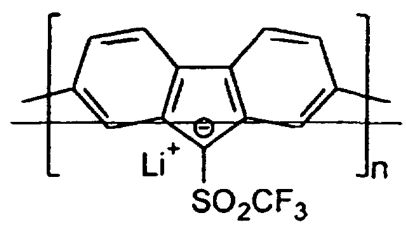

Une composition ionique de la présente invention peut également être utilisée comme électrolyte d'une supercapacité. Un autre objet de la présente invention est par conséquent une supercapacité utilisant au moins une électrode de carbone à haute surface spécifique, ou une électrode contenant un polymère rédox, dans laquelle l'électrolyte est une composition ionique selon la présente invention.

Le cation du sel de la composition ionique utilisée comme électrolyte d'une supercapacité est de préférence un ammonium $NH_4^+$, un cation métallique, ou un cation organique comprenant un atome d'azote quaternisé. Les cations préférés sont Li, K, Ca, les terres rares, et les ions imidazolium, triazolium, pyridinium, ou 4-diméthyl aminopyridinium.

Le sel de la composition ionique de l'invention utilisée comme électrolyte d'une supercapacité est avantageusement choisi parmi les sels dans lesquels R, R' et $X_F$ ont un faible nombre d'atome, de préférence inférieur à 3. Les composés dans lesquels R et R' représentent $-CH_3$ ou $-N(CH_3)_2$ et $X_F$ représente $CF_3$- sont particulièrement préférés. Il peut être avantageux d'ajouter une faible proportion de composés ioniques dans lesquels R et/ou R' sont de longues chaînes alkyles ($n \geq 8$) pour agir en tant que tensio-actif et améliorer la pénétration du liquide dans les micropores du carbone de l'électrode de carbone.

Les solvants sont de préférence choisis parmi ceux qui ont une faible viscosité et qui confèrent à la composition une conductivité élevée, par exemple l'acétonitrile, les carbonates d'alkyle, le mélange carbonate d'éthylène et de diméthoxyéthane.

Une composition ionique selon la présente invention peut être utilisée comme électrolyte d'une pile à combustible. Pour cette application particulière, le sel de l'électrolyte est choisi avantageusement parmi les sels $X_F$-$SO_x$-$C^-$(Z)(Z') ou $X_F$-$SO_x$-$C^-$(Z)(YR), les premiers cités étant préférés.

Le cation du composé utilisé dans l'électrolyte d'une pile à combustible est de préférence un hydronium, un ammonium, un cation métallique, ou un cation organique comprenant un atome d'azote quaternisé (en remplacement partiel pour l'hydronium). Les cations préférés sont $H^+$, $H_3O^+$, $Li^+$, $K^+$, $Ca^{++}$, les ions imidazolium, triazolium, pyridinium et 4-diméthyl aminopyridinium.

Les substituants Z ou Z' du composé ionique sont choisis de préférence parmi $-\Phi OC_nF_{2n+1}$, $-\Phi OC_2F_4H$, $-\Phi SC_nF_{2n+1}$ et $-\Phi SC_2F_4H$, $-\Phi OCF=CF_2$, $-\Phi SCF=CF_2$, $-C_2F_4H$ et $CF_2=CF-$, n étant un nombre entier de 1 à 8. Un tel sel est un précurseur de monomères et de polymères stables, en particulier vis-à-vis de l'oxygène. L'électrolyte présente ainsi une grande stabilité, même à des températures supérieures à 80°C lorsque le sel est un polymère.

Une composition ionique de la présente invention peut être utilisée comme électrolyte dans les systèmes photoélectrochimiques, en particulier les systèmes de conversion de la lumière en électricité, et dans les systèmes de modulation de la lumière de type électrochrome. Un dispositif photoélectrochimique dans lequel l'électrolyte est une composition ionique selon l'invention est un autre objet de la présente invention.

Il est avantageux d'utiliser comme électrolyte une composition ionique dans laquelle le cation du sel est $H^+$, $H_3O^+$, $Na^+$, $K^+$, $NH_4^+$, un imidazolium, un triazolium, un pyridinium ou un 4-diméthyl aminopyridinium. Lorsque le dispositif électrophotochimique est un système de conversion de lumière en électricité, ou un système de modulation de la lumière du type électrochrome, il est avantageux d'utiliser dans l'électrolyte un sel qui porte au moins un substituant R, R' ou $R^H$ contenant un couple rédox tel qu'un disulfure, une thioamide, un ferrocène, une phéno-thiazine, un groupe bis(dialkylaminoaryle), un nitroxyde, un imide aromatique. L'électrolyte présente ainsi des propriétés de navette rédox utiles pour assurer le passage de courant sans polarisation. Lorsque le cation du composé ionique est un imidazolium, un triazolium, un pyridinium ou un 4-diméthyl aminopyridinium, le composé ionique est fondu à la température ambiante et présente une conductivité ionique élevée, supérieure à $10^{-3}$ $S.cm^{-1}$. Une composition contenant ledit sel, la base correspondante (imidazole, triazole, pyridine, 4-diméthyl aminopyridine) et un poly(oxyde d'éthylène) de préférence de masse élevée ou réticulable, constitue un polymère conducteur protonique anhydre.

Pour un dispositif électrochrome à colorants, on utilise comme électrolyte une composition selon l'invention dans laquelle le sel est un composé ayant des propriétés de sel fondu et qui contient en outre des colorants complémentaires qui changent de couleur en passant de l'état oxydé à l'état réduit et inversement.

Une composition ionique selon l'invention peut également être utilisée en tant qu'électrolyte ou colorant électroactif dans un dispositif pour l'affichage optique. Dans cette utilisation particulière, il est avantageux de choisir pour R, R' ou $R^H$, un alkyle, un aryle, un alkylaryle ou un arylalkyle, qui permet d'induire dans la composition ionique des propriétés de type mésogène. Les groupes alkyles ayant de 6 à 20 atomes de carbones et, les groupes aryle-alkyle, en particulier ceux contenant l'entité biphényle qui forment des phases de type cristal liquide, sont particulièrement préférés.

Une composition ionique de la présente invention peut également être utilisée pour le dopage p ou n d'un polymère à conduction électronique et cette utilisation constitue un autre objet de la présente invention. Les anions du sel de la composition ionique de l'invention peuvent servir de contre charge à des polymères conjugués polycationiques tels le polyacétylène, le polypyrrole, le polythiophène, le polyparaphénylène, les polyquinolines et leur copolymères alternés avec l'acétylène (ex polyparaphénylènevinylène), et leur dérivés de substitution par les groupements alkyle, alcoxy, aryle etc... Eventuellement, la polymérisation ou le dopage sont effectués par voie électrochimique à partir des compositions ioniques, ou par échange des anions d'un polymère déjà dopé par les anions des compositions ioniques.

La charge anionique portée par l'un des anions $X_F$-$SO_x$-$C^-$ (Z)(Z'), $X_F$-$SO_x$-$C^-$(YR)(Y'R') ou $X_F$-$SO_x$-$C^-$(Z) (YR) exerce un effet stabilisant sur les conducteurs électroniques de type polymères conjugués, et l'utilisation d'une composition contenant un sel portant au moins un substituant Z, Z', R ou R' comprenant une longue chaîne alkyle permet de rendre ces polymères solubles dans les solvants organiques usuels même à l'état dopé. Le greffage de ces charges sur le polymère lui-même donne des polymères dont la charge globale est cationique, qui sont solubles dans les solvants organiques et qui présentent, outre leur stabilité, des propriétés d'anticorrosion vis-à-vis des métaux, l'aluminium et les métaux ferreux. La présente invention a aussi pour objet des matériaux à conduction électronique comprenant une composition ionique de la présente invention dans laquelle la partie cationique du sel est un polycation constitué par un polymère conjugué dopé "p". Les sels préférés pour cette application sont ceux dans lesquels l'un des substituants Z, Z', R ou R' contient au moins une chaîne alkyle ayant de 6 à 20 atomes de carbone. A titre d'exemple, on peut citer les composés dans lesquels R ou R' est un radical alkyle. On peut également citer les composés dans lesquels $X_F$ est $R^HCF_2$-, $R^HCF_2CF_2$-, $R^HCF_2CF(CF_3)$- ou $CF_3C(R^H)F$- dans lesquelles $R^H$- représente un radical alkyle. On peut citer en outre les composés dans lesquels Z représente un noyau aromatique portant un radical alkyle.

Il a été observé que la forte dissociation des espèces ioniques des composés de l'invention se traduisait par une stabilisation des carbocations, en particulier ceux dans lesquels il existe une conjugaison avec l'oxygène ou l'azote et, d'une manière surprenante, par une forte activité de la forme protonée des composés l'invention sur certains monomères. La présente invention a donc également pour objet l'utilisation d'une composition ionique comme initiateur photochimique ou thermochimique source d'acide de Brønsted catalyseur de polymérisation ou de réticulation de monomères ou de prépolymères capables de réagir par voie cationique, comme catalyseur pour diverses réactions chimiques ou comme catalyseur pour la modification de polymères. Comme catalyseur pour la modification chimique de polymères, on préfère tout particulièrement les compositions dans lesquelles le cation du sel est un proton, un oxonium, Li, Mg, Cu, une terre rare, le triméthylsilyle, un ferrocène, un zirconocène, ou un zirconoindocène.

Le procédé de polymérisation ou de réticulation de monomères ou de prépolymères capables de réagir par voie cationique est caractérisé en ce que l'on utilise une composition ionique de l'invention comme photoinitiateur source d'acide catalysant la réaction de polymérisation. Les compositions ioniques selon l'invention dans lesquelles le cation du sel est un groupe possédant une liaison $\sim N{\equiv}N^+$ ou -N=N-, un groupement sulfonium, un groupement

iodonium, ou un cation arène-ferrocénium substitué ou non, éventuellement incorporé dans une trame polymérique, sont préférées. Les compositions contenant un sel de 2,2'[Azobis(2-2'-imidazolinio-2-yl)propane]$^{2+}$ ou 2,2'-Azobis (2-amidiniopropane)$^{2+}$ sont particulièrement adaptées en tant qu'initiateurs photothermiques. Les compositions contenant un sel d'iodonium sont particulièrement adaptées en tant qu'initiateur photochimique. A titre d'exemple de sels, on peut citer

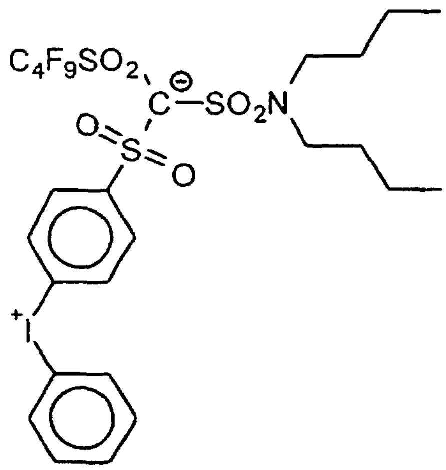

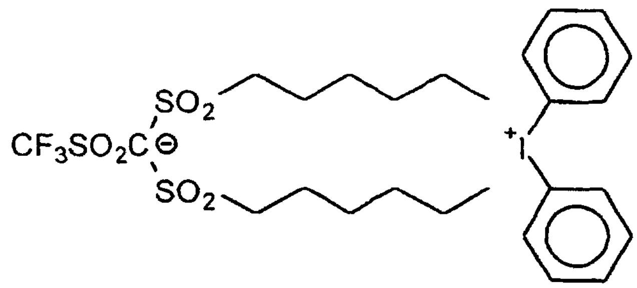

Le choix du substituant $X_F$ d'une part, des substituants R, R' ou Z d'autre part, est effectué de manière à augmenter la compatibilité du sel avec les solvants utilisés pour la réaction des monomères ou des prépolymères, et en fonction des propriétés souhaitées pour le polymère final. Par exemple, le choix de radicaux alkyles non substitués donne une solubilité dans les milieux peu polaires. Le choix de radicaux comprenant un groupe oxa ou une sulfone donnera une solubilité dans les milieux polaires. Les radicaux incluant un groupement sulfoxyde, un groupement sulfone, un groupement oxyde de phosphine, un groupement phosphonate, obtenus respectivement par addition d'oxygène sur les atomes de soufre ou de phosphore, peuvent conférer au polymère obtenu des propriétés améliorées en ce qui concerne l'adhésion, la brillance, la résistance à l'oxydation ou aux UV. Les monomères et les prépolymères qui peuvent être polymérisés ou réticulés à l'aide des photoinitiateurs de la présente invention sont ceux qui peuvent subir une polymérisation cationique.

Parmi les monomères, on peut citer les monomères qui comportent une fonction éther cyclique, une fonction thioéther cyclique ou une fonction amine cyclique, les composés vinyliques, (plus particulièrement les éthers vinyliques), les oxazolines, les lactones et les lactames.

Parmi les prépolymères, on peut citer les composés dans lesquels des groupements époxy sont portés par une chaîne aliphatique, une chaîne aromatique, ou une chaîne hétérocyclique, par exemple les éthers glycidiques du bisphénol A éthoxylés par 3 à 15 unités d'oxyde d'éthylène, les siloxanes possédant des groupements latéraux du type époxycyclohexène-éthyle obtenus par hydrosilylation des copolymères de dialkyl, d'alkylaryle ou de diaryl siloxane avec le méthyl hydrogénosiloxane en présence d'oxyde de vinylcyclohexène, les produits de condensation du type sol-gel obtenus à partir du triéthoxy ou du triméthoxy silapropylcyclohexène oxyde, les uréthanes incorporant les produits de réaction du butanediol monovinyléther et d'un alcool de fonctionnalité supérieure ou égale à 2 sur un di ou un tri isocyanate aliphatique ou aromatique.

Le procédé de polymérisation selon l'invention consiste à mélanger au moins un monomère ou prépolymère capable de polymériser par voie cationique et au moins une composition ionique de l'invention, et à soumettre le mélange obtenu à un rayonnement actinique ou un rayonnement β. De préférence, le mélange réactionnel est soumis au rayonnement après avoir été mis sous forme d'une couche mince ayant une épaisseur inférieure à 5 mm, de préférence sous forme d'un film mince ayant une épaisseur inférieure ou égale à 500 µm. La durée de la réaction dépend de l'épaisseur de l'échantillon et de la puissance de la source à la longueur d'onde λ active. Elle est définie par la vitesse de défilement devant la source, qui est comprise entre 300 m/min et 1 cm/min. Des couches de matériau final ayant une épaisseur supérieure à 5 mm peuvent être obtenues en répétant plusieurs fois l'opération consistant à épandre une couche et à la traiter par le rayonnement.

Généralement, la quantité de composition agissant comme initiateur photochimique utilisée est comprise entre 0,01 et 15 % en poids par rapport au poids de monomère ou de prépolymère, de préférence entre 0,1 et 10 % en poids.

Une composition ionique de la présente invention contenant une quantité très faible, voire nulle, de solvant peut être utilisée comme initiateur photochimique, notamment lorsque l'on souhaite polymériser des monomères liquides dans lesquels le sel de la composition ionique utilisée comme initiateur photochimique est soluble ou aisément dispersable. Cette forme d'utilisation est particulièrement intéressante, car elle permet de supprimer les problèmes liés aux solvants (toxicité, inflammabilité).

Une composition ionique de la présente invention peut également être utilisée en tant qu'initiateur photochimique ou thermochimique sous forme d'une solution homogène dans un solvant inerte vis-à-vis de la polymérisation, prête à l'emploi et aisément dispersable, en particulier dans le cas où le milieu à polymériser ou à réticuler présente une viscosité élevée.

Comme exemple de solvant inerte, on peut citer les solvants volatils, tels que l'acétone, la méthyl-éthyl cétone et l'acétonitrile. On peut également citer les solvants non volatils tels que par exemple le carbonate de propylène, la $\gamma$-butyrolactone, les éther-esters des mono-, di-, tri- éthylène ou propylène glycols, les éther-alcools des mono-, di-, tri- éthylène ou propylène glycols, les diéthers des mono-, di- ou triéthylène glycol et les diéthers des mono-, di- ou tripropylène glycol, et les plastifiants tels que les esters de l'acide phtalique ou de l'acide citrique.

Pour irradier le mélange réactionnel, le rayonnement peut être choisi parmi le rayonnement ultraviolet, le rayonnement visible, les rayons X, les rayons $\gamma$ et les rayons $\beta$. Lorsque l'on utilise la lumière ultraviolette comme rayonnement actinique, il peut être avantageux d'ajouter aux initiateurs photochimique ou thermochimiques de l'invention des photosensibilisateurs destinés à permettre une photolyse efficace avec les longueurs d'ondes moins énergétiques que celles correspondant au maximum d'absorption du initiateur photochimique ou thermochimique, telles que celles émises par les dispositifs industriels, ($\lambda \approx 300$ nm pour les lampes à vapeur de mercure en particulier).

Parmi les différents types de rayonnement mentionnés, le rayonnement ultraviolet est particulièrement préféré. D'une part, il est plus commode d'emploi que les autres rayonnements mentionnés. D'autre part, les initiateurs photochimiques sont en général directement sensibles aux rayons UV et les photosensibilisateurs sont d'autant plus efficaces que la différence d'énergie ($\delta\lambda$) est plus faible.

Les composés ioniques de l'invention peuvent aussi être mis en oeuvre en association avec des amorceurs de type radicalaire générés thermiquement ou par action d'une radiation actinique. Il est ainsi possible de polymériser ou de réticuler des mélanges de monomères ou de prépolymères contenant des fonctions dont les modes de polymérisation sont différents, par exemple des monomères ou des prépolymères polymérisant par voie radicalaire et des monomères ou des prépolymères polymérisant par voie cationique. Cette possibilité est particulièrement avantageuse pour créer des réseaux interpénétrés ayant des propriétés physiques différentes de celles qui seraient obtenues par simple mélange des polymères issus des monomères correspondants.

L'invention a également pour objet l'utilisation des compositions ioniques de l'invention pour les réactions d'amplification chimique de photoresists pour la micro-lithographie. Lors d'une telle utilisation, un film d'un matériau comprenant un polymère et une composition ionique de l'invention est soumis à une irradiation. L'irradiation provoque la formation de l'acide par remplacement du cation M par un proton, qui catalyse la décomposition ou la transformation du polymère. Après décomposition ou transformation du polymère sur les parties du film qui ont été irradiées, les monomères formés ou le polymère transformé sont éliminés et il reste une image des parties non exposées. Pour cette application particulière, il est avantageux d'utiliser une composition de l'invention qui se présente sous la forme d'un polymère constitué essentiellement d'unités récurrentes styrényles portant un substituant ionique $X_F$-$SO_x$-$C^-$ dans lequel la partie cationique est un iodonium ou un sulfonium. Ces composés permettent d'obtenir après photolyse des produits qui ne sont pas volatils, et donc ni corrosifs, ni odorants lorsqu'il s'agit de sulfures produits de la décomposition du sulfonium. Parmi les polymères qui peuvent ainsi être modifiés en présence d'un composé de l'invention, on peut citer notamment les polymères contenant des motifs ester ou des motifs aryléther de tertioalkyle, par exemple les poly-(phtalaldéhydes), les polymères de bisphénol A et d'un diacide, le polytertiobutoxycarbonyl oxystyrène, le poly-tertiobutoxy-$\alpha$-méthyl styrène, le polyditertiobutylfumarate-co-allyltriméthylsilane et les polyacrylates d'un alcool tertiaire, en particulier le polyacrylate de tertiobutyle. D'autres polymères sont décrits dans J.V. Crivello et al, Chemistry of Materials 8, 376-381, (1996).

Dans les sels des compositions de la présente invention, les paires d'ions présentent un très forte dissociation, ce qui permet l'expression des propriétés catalytiques intrinsèques du cation $M^{m+}$, dont les orbitales actives sont facilement exposées aux substrats de la réaction, ceci dans des milieux variés. La plupart des réactions importantes de la chimie organique peuvent être ainsi effectuées dans des conditions peu contraignantes, avec d'excellents rendements et la facilité de séparer le catalyseur du milieu réactionnel. La mise en évidence d'induction asymétrique par l'utilisation d'une composition ionique selon l'invention contenant un sel qui porte un groupement chiral est particulièrement importante de part sa généralité et sa facilité de mise en oeuvre. Il est à noter que les molécules perfluorées chirales $[(R_FSO_2)_3C]^-$, $1/mM^{m+}$ sont inconnues et ne présenteraient qu'une activité optique négligeable du fait de la faible polarisabilité des groupements perfluorés. La présente invention a par conséquent comme autre objet l'utilisation des compositions de l'invention comme catalyseurs dans les réactions de Friedel-Crafts, les réactions de Diels-Alder, les réactions d'aldolisation, les additions de Michael, les réactions d'allylation, les réactions de couplage pinacolique, les réactions de glycosylation, les réactions d'ouvertures de cycles des oxétanes, les réactions de métathèse des alcènes, les polymérisations de type Ziegler-Natta, les polymérisations du type métathèse par ouverture de cycle et

les polymérisations du type métathèse de diènes acycliques. Les compositions ioniques de l'invention préférées pour une utilisation en tant que catalyseur pour les réactions ci-dessus sont celles dans lesquelles le cation du sel est choisi parmi H, le lithium, le magnésium, les métaux de transition à l'état divalent ou trivalents, les terres rares, les platinoïdes, et leurs couples organométalliques, en particulier les métallocènes. Les compositions contenant un sel ayant un substituant R, R' ou Z optiquement actif sont particulièrement efficaces pour la catalyse énantiosélective.

Les compositions de l'invention peuvent également être utilisées comme solvant pour effectuer des réactions chimiques, photochimiques, électrochimiques, photoélectrochimiques. Pour cette utilisation particulière, on préfère les compositions ioniques dans lesquelles le sel a un cation du type imidazolium, triazolium, pyridinium ou 4-diméthylamino-pyridinium, ledit cation portant éventuellement un substituant sur les atomes de carbone du cycle, et un anion dans lequel les substituants ont un nombre d'atomes de carbone faible, de préférence inférieur ou égal à 4. Parmi ces sels, on préfère tout spécialement ceux qui ont un point de fusion inférieur à 150°C, plus particulièrement inférieur à 100°C. La quantité de solvant peut alors être minimisée. On peut également utiliser une composition contenant un sel d'un cation métallique solvaté, par exemple par un polyéthylène glycol ayant de préférence un masse inférieure à 1000.

Les colorants de type cationique (cyanines) sont utilisés de plus en plus fréquemment comme sensibilisateurs de films photographiques, pour le stockage optique d'information (disques optiques accessibles en écriture), pour les lasers. La tendance de ces molécules conjuguées à s'empiler lorsqu'elles sont en phases solides limite leur utilisation, par suite des variations des propriétés optiques par rapport à la molécule isolée. L'utilisation de compositions ioniques de l'invention pour la fabrication de colorants cationiques dont les contre-ions, éventuellement fixés à cette même molécule, correspondent aux fonctionnalités de l'invention, permet de réduire les phénomènes d'agrégation, y compris dans des matrices solides polymères et de stabiliser ces colorants. La présente invention a pour autre objet l'utilisation d'une composition ionique selon l'invention comme composition de colorant cationique. Les compositions ioniques particulièrement préférées pour cette application sont celles qui contiennent un sel dans lequel la ou les charges négatives du groupement anionique $X_F$-$SO_x$-$C^-$ (Z)(Z'), $X_F$-$SO_x$-$C^-$(YR)(Y'R') ou $X_F$-$SO_x$-$C^-$(Z)(YR) soit sont fixées à la molécule de colorant, soit constituent le contre-ion des charges positives du colorant. A titre d'exemple de tels composés, on peut citer les composés suivants :

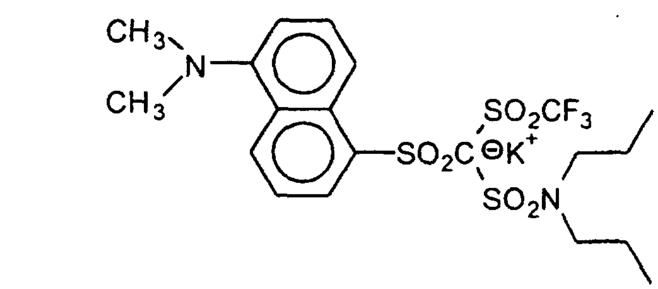

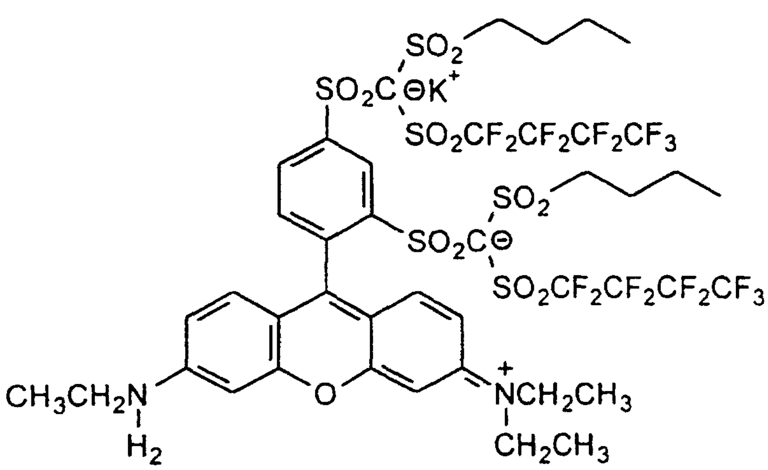

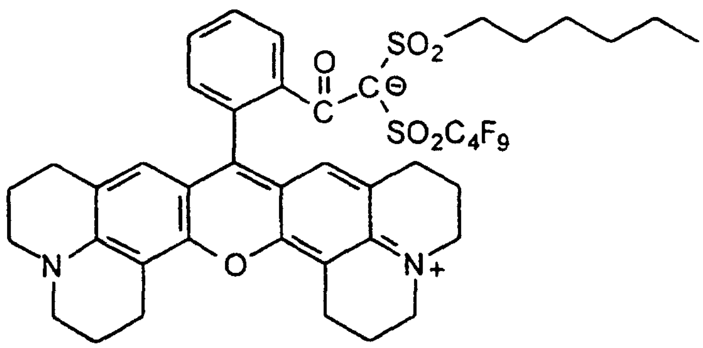

La présente invention est décrite plus en détail par les exemples suivants.

**Exemple 1**

100 mmoles de bis(chlorosulfonyl)méthane $(ClSO_2)_2CH_2$ préparé suivant la méthode de Fild et Rieck (Chem. Ztg., 100, 1976, 391) ont été ajoutés lentement à 200 ml d'une solution de diméthylamine 5M. Après deux heures sous agitation, l'eau a été évaporée et le résidu repris dans l'éther. Après évaporation de l'éther, le bis(diméthylaminosulfonyl) méthane $((CH_3)_2NSO_2)_2CH_2$ a été purifié par sublimation. Dans 20 ml de tétrahydrofurane, on a alors ajouté 20 mmoles de $((CH_3)_2NSO_2)_2CH_2$ et 20 mmoles d'hydrure de sodium par portions. Après une heure, on a ajouté 20 mmoles de trifluorométhanesulfonylimidazole (commercialisé par Fluka). Après 24 heures sous agitation, le solvant a été évaporé, puis le résidu repris dans l'éthanol et l'on a ajouté 25 mmoles de $K_2CO_3$. Après filtration et évaporation de la suspension, le sel de potassium $[(CH_3)_2NSO_2]_2C(K)SO_2CF_3$ a été recristallisé dans l'eau. Ce sel a ensuite été traité par une quantité stoechiométrique de chlorure de lithium dans le tétrahydrofurane. On a ainsi obtenu le sel de lithium correspondant :

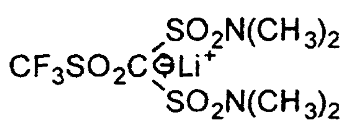

Ce sel présente une excellente solubilité dans les solvants organiques usuels (tétrahydrofurane, acétonitrile, diméthylformamide, acétate d'éthyle, glymes,...) et dans les polymères solvatants aprotiques comme le poly(oxyde d'éthylène). Une solution du sel dans le poly(oxyde d'éthylène) à une concentration O/Li = 12/1 présente une conductivité ionique supérieure à $10^{-4}$ S.cm$^{-1}$ à 60°C. Une solution concentrée de ce sel dans l'acétone peut être utilisée pour la catalyse de réactions de Diels-Alder.

On a réalisé un générateur suivant la technologie lithium-polymère en utilisant une anode en lithium métallique, un électrolyte composé d'un terpolymère d'oxyde d'éthylène, d'allylglycidyléther et de méthylglycidyléther contenant ce sel de lithium à une concentration O/Li = 20/1 et une cathode composite à base d'oxyde de vanadium (40% en volume), de noir de carbone (5% en volume) et d'un électrolyte identique à celui décrit ci-dessus (50% en volume). Ce générateur a donné un profil de cyclage à 60°C équivalent à celui obtenu en utilisant la bis(trifluorométhanesulfonyl) imide de lithium (LiTFSI) qui est l'un des sels les plus courants pour cette application.

**Exemple 2**

Dans 20 ml de tétrahydrofurane anhydre, on a ajouté 20 mmoles de triflinate de potassium $CF_3SO_2K$ (commercialisé par Parish Chemicals, Utah, USA) et 20 mmoles de 4-bromo-1-bromométhylènebenzène $BrCH_2C_6H_4Br$. Après 24 heures sous agitation, il s'est formé un précipité de bromure de potassium qui a été éliminé par filtration. On a alors ajouté par portions 40 mmoles d'hydrure de sodium NaH et, après une heure sous agitation, un catalyseur tétrakis(triphénylphosphine)-Pd$^0$ (2% molaire). Le milieu réactionnel a alors été agité pendant 48 heures, puis précipité dans une solution aqueuse saturée en chlorure de potassium. On a alors obtenu le produit suivant :

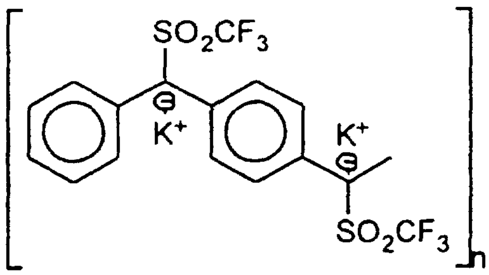

10 mmoles du polymère obtenu ont ensuite été dopés par traitement par 2,5 mmoles de diacétatoiodosobenzène $(CH_3CO_2)_2IC_6H_5$ (commercialisé par Lancaster) dans 10 ml de diméthylformamide. Après filtration et évaporation du solvant, on a obtenu un polymère à conduction électronique (PCE) à dopage anionique présentant une conductivité de l'ordre de 6 $S.cm^{-1}$, déterminée par une mesure quatre points. Cette conductivité présente une bonne stabilité en milieu humide.

Le composé polymère du présent exemple est un bon inhibiteur de corrosion de l'aluminium et des métaux ferreux en milieu acide ou chlorure. Le traitement des surfaces à protéger s'effectue simplement par dépôt d'une solution du PCE dans un mélange d'eau et de diméthylformamide, sous la forme d'une peinture, puis séchage et un traitement thermique à 100°C. Ce composé polymère a également permis d'obtenir des dépôts conducteurs adhérents dont la conductivité est stable à l'air, sur des plastiques traités par effet Corona.

Le composé ionique polymère du présent exemple peut également être utilisé pour l'élaboration de la cathode d'une batterie. La cathode est une cathode composite contenant le sel polymère obtenu dans le présent exemple (40% en volume) et un poly(oxyde d'éthylène) de masse $3.10^5$. L'électrolyte est un film de poly(oxyde d'éthylène) de masse $5.10^6$ contenant le sel de lithium de la bis(trifluorométhanesulfonyl)imide, à une concentration O/Li = 20/1. L'anode est du lithium métallique. Après avoir assemblé l'ensemble dans un boîtier de pile bouton, cette batterie a été cyclée à une température de 60°C entre 2 V et 3,9 V. Plus de 500 cycles de charge/décharge ont pu être effectués en conservant 70% de la capacité du premier cycle.

**Exemple 3**

Dans 20 ml de pyridine anhydre, on a ajouté 20 mmoles de triflinate de potassium $CF_3SO_2K$ et 20 mmoles de 1-diméthylaminosulfonyl-1-bromométhane $Me_2NSO_2CH_2Br$ préparé à partir de $ClSO_2CH_2Br$ (commercialisé par Lancaster). Après 24 heures sous agitation, la pyridine a été évaporée et le résidu repris dans 30 ml d'une solution d'acide chlorhydrique 4M. On a ensuite extrait la phase aqueuse par de l'éther, on l'a séchée sur sulfate de magnésium, puis on a évaporé l'éther et on a sublimé le résidu. On a ainsi récupéré le composé $Me_2NSO_2CH_2SO_2CF_3$. Ce composé a été agité en présence de phosphate de potassium $K_3PO_4$ dans le tétrahydrofuranne pendant 24 heures. Puis, après filtration et évaporation du solvant, on a obtenu le sel de potassium de $Me_2NSO_2CH_2SO_2CF_3$. Dans 15 ml de tétrahydrofurane, on a introduit 10 mmoles de ce sel de potassium, puis 10 mmoles de chlorure de styrènesulfonyle (commercialisé par Monomer-Polymer & Dajac Laboratories). Après 24 heures, on a ajouté 10 mmoles de chlorure de lithium, puis on a agité le milieu réactionnel pendant 6 heures. Après filtration et évaporation du solvant, on a obtenu le produit suivant:

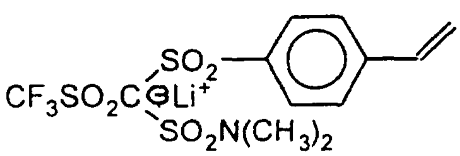

On a mis en solution dans 20 ml de tétrahydrofurane anhydre, 6 mmoles de ce sel, 4 mmoles d'acrylonitrile et 100 µmoles de 1,1'-azobis(cyclohexanecarbonitrile) et on a dégazé par un balayage d'argon sec. On a alors effectué sous argon la copolymérisation de l'acrylonitrile avec le dérivé du styrène en chauffant le milieu réactionnel à 60°C pendant 48 heures. Après refroidissement, la solution a été concentrée, puis le polymère récupéré par reprécipitation dans l'éther. On a ainsi obtenu le copolymère suivant :

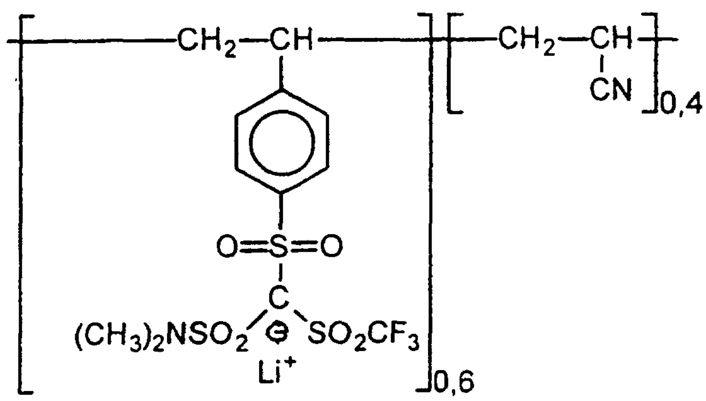

On a introduit dans ce copolymère du carbonate d'éthylène (EC) et du carbonate de propylène (PC) dans les proportions suivantes, exprimées en pourcentage en poids : copolymère 30 %, EC 35 %, PC 35 % et on a obtenu un polyélectrolyte sous forme de gel. Ce gel présente de bonnes propriétés mécaniques et une conductivité supérieure à $10^{-4}$ $S.cm^{-1}$ à 30°C. Le nombre de transport cationique dans cet électrolyte est de 0,85.

On a assemblé un générateur électrochimique en utilisant comme anode un coke de carbone (80% en volume) mélangé avec le copolymère du présent exemple (20% en volume). L'électrolyte est le copolymère gel décrit ci-dessus. La cathode est une cathode composite contenant du noir de carbone (6% en volume), $LiNiO_2$ (75% en volume) et le copolymère du présent exemple (20% en volume). Ce générateur a donné de bonnes performances en cyclage à 25°C. Après 500 cycles de charge/décharge entre 3 et 4,2 V, une capacité supérieure à 75% de la capacité au premier cycle a été constatée. Le générateur présente en outre de très bonne performances lors d'appel de puissance du fait de l'utilisation d'anions fixés, qui a également amélioré l'évolution de la résistance d'interface.

Suivant un procédé équivalent, on a également synthétisé un polyanion contenant 3% molaire du sel de lithium et 97% molaire d'acrylonitrile. Ce copolymère a permis d'induire des propriétés antistatiques dans des fibres de polyacrylonitrile.

**Exemple 4**

En opérant comme dans l'exemple 1, mais en remplaçant la diméthylamine par de la dibutylamine, on a préparé le sel de potassium $[(C_4H_9)_2NSO_2]_2CKSO_2CF_3$. 5 mmoles de ce sel de potassium et 5 mmoles de chlorure de diphényliodonium $(C_6H_5)_2ICl$ ont ensuite été agités ensemble durant 24 heures dans l'eau. Après extraction de la phase aqueuse par du dichlorométhane, évaporation du dichlorométhane et séchage, on a récupéré le produit suivant :

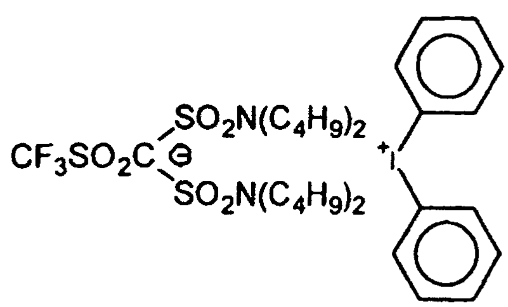

Ce sel permet d'amorcer sous l'effet d'un rayonnement actinique (lumière, rayons $\gamma$, faisceaux d'électrons), la réaction de réticulation cationique de monomères riches en électrons (éthers vinyliques, alkyl vinyl éthers,...). Il est soluble dans la plupart des solvants organiques usuels (tétrahydrofurane, acétonitrile, diméthylformamide, acétate d'éthyle, glymes,...) et dans les polymères solvatants aprotiques comme le poly(oxyde d'éthylène). Il est aussi soluble à plus de 10% en poids dans les solvants réactifs comme le triéthylèneglycol divinyl éther, contrairement par exemple au sel de bis(trifluorométhanesulfonyl)imide du diphényliodonium. Ce sel a été utilisé comme initiateur photochimique pour la polymérisation de triéthylèneglycol divinyl éther. On a mis en solution 1% en poids du sel dans le triéthylèneglycol divinyl éther, puis on a irradié par un rayonnement U.V. à 254 nm, d'une puissance de 1900 $mW/cm^2$. Après quelques secondes, le solvant réactif a pris en masse, la réaction étant très exothermique.

**Exemple 5**

50 mmoles du sel de potassium $((CH_3)_2NSO_2)_2CKSO_2CF_3$, préparé selon le mode opératoire de l'exemple 1, ont été cobroyés dans un mortier en agate en boîte à gants avec 17,27 g (150 mmoles) d'hydrogénosulfate d'ammonium $HSO_4NH_4$ (commercialisé par Aldrich). Par sublimation sous vide secondaire à 80°C, on a récupéré la forme acide $[(CH_3)_2NSO_2]_2CHSO_2CF_3$.

A une solution dans 15 ml d'éther de 20 mmoles d'imidazole, on a ajouté 20 mmoles de $[(CH_3)_2NSO_2]_2CHSO_2CF_3$, puis, après agitation pendant 24 heures, évaporation de l'éther et séchage, on a obtenu le sel d'imidazolium :

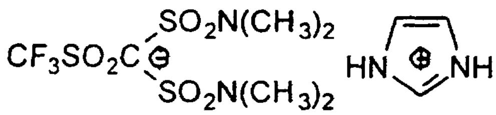

Le broyage en boîte à gants d'un mélange d'imidazole et du sel d'imidazolium dans un rapport molaire 1/2 a permis d'obtenir une composition ayant une température de fusion inférieure à l'ambiante. Ce sel fondu présente une conductivité protonique élevée, supérieure à $10^{-3}$ S.cm$^{-1}$ à 60°C. L'addition au sel fondu de poly(oxyde d'éthylène), préférentiellement de haute masse ou pouvant être ultérieurement réticulé, permet d'obtenir un électrolyte polymère conducteur protonique anhydre, sans perte de conductivité.

Un tel électrolyte polymère est particulièrement intéressant pour la réalisation de systèmes de modulation de la lumière tels que les vitrages électrochromes ou les systèmes électrochromes à colorants. On a ainsi obtenu une membrane optiquement transparente dans le visible et ayant une bonne tenue mécanique en utilisant un électrolyte polymère constitué par 80 % en poids du sel fondu ci-dessus et par 20% en poids de poly(oxyde d'éthylène) de masse $M_w = 5.10^6$. On a ensuite réalisé, en boîte à gants, un système électrochrome utilisant cet électrolyte enfermé entre une première électrode constituée par le dépôt sur une plaque de verre d'une couche d'oxyde d'iridium hydrogéné $H_xIrO_2$ et d'une sous couche conductrice d'oxyde d'étain et une seconde électrode constituée d'une couche de trioxyde de tungstène $WO_3$ et d'une sous-couche conductrice d'oxyde d'étain. Cet système électrochrome permet une variation de l'absorption optique variant de 80% (état décoloré) à 30% (état coloré) et de bonnes performances en cyclage. Plus de 20 000 cycles coloration/décoloration ont été réalisés.

On a également réalisé un système électrochrome en dissolvant deux colorants complémentaires dans le sel fondu décrit ci-dessus. Dans une boîte à gants, on a co-broyé 1,62 g (5 mmoles) du sel d'imidazolium du (trifluorométhanesulfonyl)[bis(diméthylaminosulfonyl)]méthide avec 1,02 g d'imidazole (15 mmoles). Puis on a ajouté 16,5 mg (50 μmoles) de la forme leuco du vert malachite (état réduit incolore) et 29,5 mg (50 μmoles) du sel de 3-(4,5-diméthylthiazolyl-2-yl)-2,5-diphényl-2H-tétrazolium (MTT) de trifluorométhanesulfonyl(diméthylaminosulfonyl)imide (état oxydé incolore, obtenu par échange ionique dans l'eau à partir du bromure). On a alors ajouté 5% en poids de poly(oxyde d'éthylène) de masse $M_w = 3.10^5$. Le gel obtenu a été déposé entre deux plaques de verre recouvertes d'une couche conductrice d'oxyde d'étain ($SnO_2$). Après pressage sous vide pour homogénéiser le dépôt et scellage pour le rendre étanche, on a obtenu un système électrochrome à colorants. On a appliqué au système un potentiel de 1300 mV à l'aide d'un potentiostat. Le système s'est alors coloré, la forme oxydée de la malachite verte et la forme réduite du MTT présentant chacune une bande d'absorption intense dans le visible. En appliquant un potentiel de -500 mV, on a observé une décoloration relativement rapide du système (inférieur à 60 s). Un tel système électrochrome est facile à mettre en oeuvre, même pour des dispositifs de grande taille (supérieure au $m^2$) qui utilisent aussi bien du verre qu'un polymère convenablement traité comme électrode transparente conductrice. De plus, l'énergie nécessaire pour conserver la coloration est relativement faible, inférieure à 1 W/$m^2$.

**Exemple 6**

20 mmoles du sel de potassium $((CH_3)_2NSO_2)_2CKSO_2CF_3$, préparé selon le mode opératoire de l'exemple 1 et 22 mmoles de chlorure de 1-éthyl-3-méthyl-1*H*-imidazolium (10% en excès, commercialisé par Aldrich) ont été mélangées dans l'eau. On a obtenu une phase liquide plus dense que l'eau qui a été récupérée par extraction au dichlorométhane. Après évaporation du dichlorométhane et séchage sous vide à 40°C du liquide obtenu, on a obtenu le sel fondu suivant :

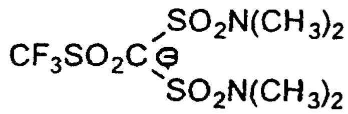 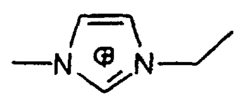

Ce sel fondu présente une conductivité supérieure à $10^{-3}$ $S.cm^{-1}$ et un point de congélation inférieur à 25°C. Son large domaine de stabilité rédox en fait un électrolyte particulièrement intéressant pour les générateurs électrochimiques tels que les batteries au lithium, les super-capacités, les systèmes de modulation de la lumière, les cellules photovoltaïques.

Une cellule photovoltaïque électrochimique similaire dans le principe à celle décrite dans le brevet Européen EP 613466 a été réalisée en assemblant un système composé de deux électrodes séparées par un espace vide d'une épaisseur de 30 µm. La première électrode a été revêtue d'une couche nanoparticulaire de dioxyde de titane $TiO_2$ de 0,25 µm d'épaisseur sur laquelle a été adsorbée la 1,3-phénylsulfonyl-(trifluorométhanesulfonyl-diméthylaminosulfonyl)méthane rhodamine B comme sensibilisateur. L'espace entre les électrodes a été rempli avec un électrolyte composé du sel fondu dans lequel avait été introduit 10% en poids d'iodure de méthylhexyl imidazolium et 10 mmoles d'iode. Cette cellule photovoltaïque a donné des performances intéressantes, en particulier un courant de court-circuit de 103 $\mu A.cm^{-2}$ et une tension en circuit ouvert de 552 mV.

La rhodamine B modifiée utilisée ci-dessus a été obtenue de la manière suivante :

A 5 mmoles de sulforhodamine B (commercialisé par Aldrich) dans 15 ml de diméthylformamide anhydre, on a ajouté 5 mmoles du sel de potassium de l'acide trifluorométhanesulfonique $CF_3SO_3K$. Après deux heures sous agitation, on a ajouté lentement 10 mmoles de chlorure d'oxalyle ClCOCOCl en solution dans 10 ml de dichlorométhane anhydre. La réaction s'est poursuivie pendant une nuit sous argon, puis on a ajouté 20 mmoles du sel de potassium $(CH_3)_2NSO_2CHKSO_2CF_3$, préparé comme à l'exemple 3. Après 48 heures, la diméthylformamide a été évaporée et le résidu recristallisé dans 40 ml d'eau. Après filtration et séchage, on a obtenu le produit attendu :

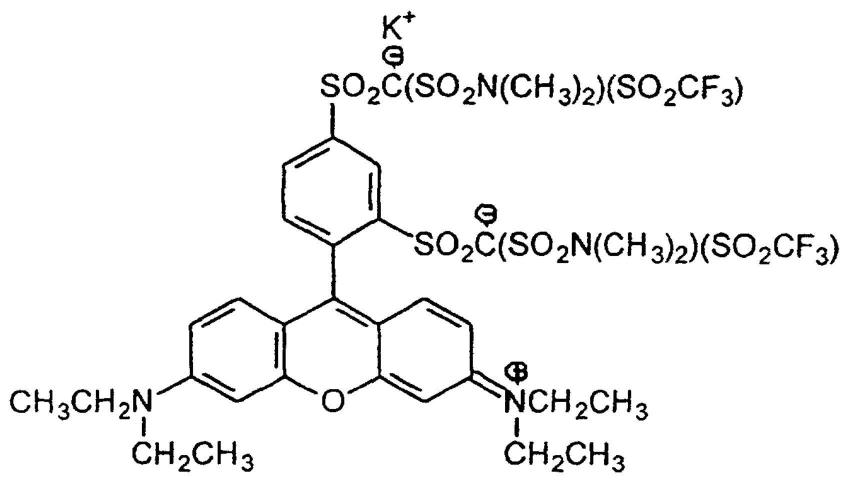

On a également réalisé une supercapacité en utilisant le sel fondu du présent exemple comme électrolyte et, comme électrodes, des électrodes composites carbone/aluminium. Les électrodes d'une épaisseur de 150 µm ont été placées de part et d'autre d'un film de polyéthylène microporeux d'une épaisseur de 40 µm et le système complet scellé en boîte à gants dans un boîtier de pile bouton. La supercapacité ainsi obtenue a permis de réaliser plus de 100 000 cycles de charge/décharge entre 0 et 2,5 V pour une densité d'énergie supérieure à 25 Wh/l et une puissance délivrée supérieure à 1500 W/l.

Le sel de lithium $[(CH_3)_2NSO_2]_2CLiSO_2CF_3$, préparé selon le mode opératoire de l'exemple 1, a été dissous dans le sel fondu du présent exemple, à une concentration de 0,5 M. La composition ainsi obtenue a été utilisée comme électrolyte dans une batterie comprenant une anode à base de titanate de lithium $Li_4Ti_5O_{12}$ et une cathode d'oxyde mixte de cobalt et de lithium $LiCoO_2$. Cette batterie a donné des performances équivalente à celles d'une batterie similaire contenant un électrolyte liquide à base du mélange carbonate de d'éthylène/carbonate de méthyle(50/50 v/v) contenant comme sel 1M $LiPF_6$.

**Exemple 7**

Le sel de lanthane $[(CH_3)_2NSO_2]_2C(1/3La^{3+})SO_2CF_3$ a été obtenu en traitant le sel de potassium obtenu dans l'exemple 1 par une quantité stoechiométrique de perchlorate de lanthane $La(ClO_4)_3$ dans l'acétonitrile. Après filtration pour éliminer le précipité de perchlorate de potassium $KClO_4$ et évaporation du solvant, on a récupéré quantitativement le sel de lanthane.

Ce sel a été utilisé comme catalyseur d'une réaction de Diels-Alder, à savoir la réaction de la méthylvinylcétone avec le cyclopentadiène, suivant une procédure similaire à celle décrite par Kobayashi, Nie & Sonoda (*Synlett,* (1996), 171-172) pour des sels de lanthane de bis(perfluorosulfonyl)imide.

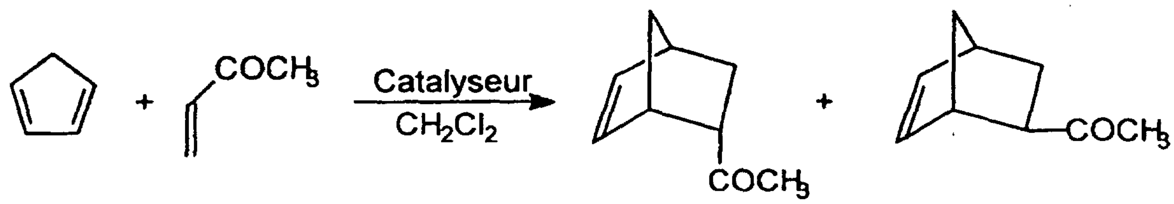


A une solution dans 10 ml de dichlorométhane de cyclopentadiène (10 mmoles) fraîchement distillé et de méthylvinylcétone, on a ajouté 200 µmoles du sel de lanthane. Après 24 heures à l'ambiante, le milieu réactionnel a été filtré pour éliminer le catalyseur en suspension. On a obtenu un rendement de réaction, déterminé par chromatographie en phase gazeuse, supérieur à 90%.

## Revendications

1. Composition ionique comprenant au moins un composé ionique en solution dans un solvant, ledit composé comprenant une partie anionique associée à au moins une partie cationique $M^{m+}$ en nombre suffisant pour assurer la neutralité électronique de l'ensemble, ladite composition étant **caractérisée en ce qu'**elle a une conductivité supérieure à $10^{-5}$ $S.cm^{-1}$ à une température comprise entre -30°C et 150°C, **en ce que** $M^{m+}$ est un proton, un hydronium, un hydroxonium, un nitrosonium $NO^+$, un ammonium $-NH_4^+$, ou un cation ayant la valence m choisi parmi les cations métalliques, les cations organiques et les cations organométalliques, et **en ce que** la partie anionique répond à l'une des formules $X_F$-$SO_x$-$C^-$ (Z)(Z'), $X_F$-$SO_x$-$C^-$(YR)(Y'R') ou $X_F$-$SO_x$-$C^-$(Z)(YR) dans lesquelles :

   - x est 1 ou 2 ;
   - $X_F$ représente un radical monovalent ou multivalent choisi dans le groupe constitué par les radicaux perhalogénés linéaires, ramifiés ou cycliques du type alkyle, alkylaryle, oxa-alkyle, aza-alkyle, thia-alkyle, alkényle, oxa-alkényle, aza-alkényle, thia-alkényle ou dialkylazo, ou dans le groupe constitué par les radicaux organiques dans lesquels le carbone en α du groupe $SO_x$ porte au moins un atome F, étant entendu qu'un radical $X_F$ multivalent est relié à plus d'un groupe $-SO_xC-$ ;
   - Z et Z' représentent indépendamment l'un de l'autre un radical électro-attracteur monovalent ou multivalent, étant entendu qu'un radical Z ou Z' multivalent peut être relié à plusieurs groupes $-C^-SO_x-X_F$, ledit radical étant choisi parmi :

     * $-NO_2$, -SCN, $-N_3$, $-CF_3$, $R'_FCH_2$- ($R'_F$ étant un radical perfluoré), $CF_2$=CFO-, $CF_2$=CF-S-, $CF_2$=CF-, $-C_2F_4H$ les radicaux fluoroalkyloxy, les radicaux perfluoroalkyloxy, les radicaux fluoroalkylthioxy et les radicaux perfluoroalkylthioxy ;
     * les radicaux comprenant un ou plusieurs noyaux aromatiques contenant éventuellement au moins un atome d'azote, d'oxygène, de soufre ou de phosphore, lesdits noyaux pouvant être éventuellement des noyaux condensés et/ou lesdits noyaux pouvant éventuellement porter au moins un substituant choisi dans le groupe constitué par les halogènes, $-OC_nF_{2n+1}$, $-OC_2F_4H$, $-SC_nF_{2n+1}$, $-SC_2F_4H$, -O-CF=$CF_2$, -SCF=$CF_2$, -CN, $-NO_2$, -SCN, $-N_3$, $-CF_3$, $CF_3CH_2$-, les radicaux aza, thia et oxa, les radicaux perfluoroalkyles, les radicaux fluoroalkyloxy, les radicaux fluoroalkylthioxy, les radicaux alkyles, alkényles, oxa-alkyles, oxa-alkényles, aza-alkyles, aza-alkényles, thia-alkyles, thia-alkényles, les radicaux polymères, et les radicaux possédant au moins un groupement ionophore cationique et/ou au moins un groupement ionophore anionique ;

étant entendu qu'un substituant Z ou Z' peut être un radical monovalent, une partie d'un radical multivalent relié à plusieurs groupements $X_F$-$SO_x$-$C^-$, ou un segment d'un polymère, et que deux substituants Z et Z' peuvent former ensemble un groupement multivalent comprenant un noyau ou plusieurs noyaux aromatiques éventuellement condensés, incluant ou non le carbone portant la charge anionique ;

- Y ou Y' représentent un groupe sulfonyle, sulfinyle, carbonyle ou phosphonyle ;
- R est un radical organique monovalent ou multivalent et R' est H ou un radical organique monovalent ou multivalent, ledit radical organique étant choisi dans le groupe constitué par les radicaux alkyle, alkényle, oxa-alkyle, oxa-alkényle, aza-alkyle, aza-alkényle, thia-alkyle, thia-alkényle et dialkylazo ayant de 1 à 24 atomes de carbone, ou par les radicaux aryle, arylalkyle, alkylaryle et alkénylaryle ayant de 5 à 24 atomes de carbone,

étant entendu que chacun des substituants R, R', Z ou Z' peut faire partie d'un polymère, et que chacun des substituants $X_F$, R, R', Z ou Z' peut être relié à un substituant $X_F$, R, R', Z ou Z' porté par un autre centre anionique.

2. Composition ionique selon la revendication 1, **caractérisée en ce que** le cation du composé ionique est un cation métallique choisi parmi les cations de métaux alcalins, les cations de métaux alcalino-terreux, les cations de métaux de transition à l'état divalent ou trivalents, et les cations de terres rares.

3. Composition ionique selon la revendication 1, **caractérisée en ce que** le cation du composé ionique est un cation organique choisi dans le groupe constitué par les cations $R''_3O^+$ (onium), $NR''_4{}^+$ (ammonium), $R''C(NR''_2)_2{}^+$ (amidinium), $C(NHR''_2)_3{}^+$ (guanidinium), $C_5R''_6N^+$ (pyridinium), $C_3R''_5N_2{}^+$ (imidazolium), $C_2R''_4N_3{}^+$ (triazolium), $C_3R''_7N_2{}^+$ (imidazolinium), $SR''_3{}^+$ (sulfonium), $PR''_4{}^+$ (phosphonium), $IR''_2{}^+$ (iodonium), $(C_6R''_5)_3C^+$ (carbonium), les radicaux R" représentant de manière indépendante H ou un radical organique non perfluoré.

4. Composition ionique selon la revendication 3, **caractérisée en ce que** les radicaux R", identiques ou différents, sont choisis dans le groupe constitué par :

- le proton, les radicaux alkyles, alkényles, oxa-alkyles, oxa-alkényles, aza-alkyles, aza-alkényles, thia-alkyles, thia-alkényles, sila-alkyles éventuellement hydrolysables, sila-alkényles éventuellement hydrolysables et les radicaux dialkylazo, lesdits radicaux pouvant être linéaires, ramifiés ou cycliques ;
- les radicaux cycliques ou hétérocycliques comprenant éventuellement au moins une chaîne latérale comprenant des hétéroatomes tels que l'azote, l'oxygène ou le soufre ;
- les radicaux aryles, arylalkyles, alkylaryles et alkénylaryles, comprenant éventuellement des hétéroatomes dans le noyau aromatique ou dans un substituant ;
- les groupes comprenant plusieurs noyaux aromatiques ou hétérocycliques, condensés ou non, contenant éventuellement au moins un atome d'azote, d'oxygène, de soufre ou de phosphore.

5. Composition ionique selon la revendication 3, **caractérisée en ce que** le cation porte au moins deux radicaux R" différents de H, ces radicaux formant éventuellement ensemble un cycle aromatique ou non, englobant ou non le centre portant la charge cationique.

6. Composition ionique selon la revendication 3, **caractérisée en ce que** le groupe cationique est polycationique.

7. Composition ionique selon la revendication 3, **caractérisée en ce que** le cation est un hétérocycle cationique à caractère aromatique, comportant au moins un atome d'azote quaternisé dans le cycle.

8. Composition ionique selon la revendication 7, **caractérisée en ce que** le cation est choisi dans le groupe constitué par l'imidazolium, le triazolium, le pyridinium et le 4-diméthylamino-pyridinium, et par lesdits cations portant éventuellement un substituant sur les atomes de carbone du cycle.

9. Composition ionique selon la revendication 3, **caractérisée en ce que** le cation possède une liaison -N=N- ou $\sim N{\equiv}N^+$, un groupement sulfonium, un groupement iodonium, ou un cation arène-ferrocénium substitué ou non, éventuellement incorporé dans une trame polymérique.

10. Composition ionique selon la revendication 3, **caractérisée en ce que** le cation comprend un groupement 2,2' [Azobis(2-2'-imidazolinio-2-yl)propane]$^{2+}$ ou 2,2'-Azobis(2-amidiniopropane)$^{2+}$.

11. Composition ionique selon la revendication 1, **caractérisée en ce que** $X_F$ est un radical alkyle perhalogéné ayant

de 1 à 12 atomes de carbone, ou un radical alkylaryle perhalogéné ayant de 6 à 9 atomes de carbone, lesdits radicaux pouvant comporter un hétéroatome 0, N ou S entre deux atomes de carbone ou à l'extrémité du radical.

**12.** Composition ionique selon la revendication 1, **caractérisée en ce que** $X_F$ est choisi dans le groupe constitué par $R^HCF_2$-, $R^HCF_2CF_2$-, $R^HCF_2CF(CF_3)$- et $CF_3C(R^H)F$-, $R^H$ représentant un radical organique non perhalogéné.

**13.** Composition ionique selon l'une des revendications 1 ou 12, **caractérisée en ce que** $R^H$ est choisi dans le groupe constitué par les radicaux alkyle, alkényle, oxa-alkyle, oxa-alkényle, aza-alkyle, aza-alkényle, thia-alkyle, thia-alkényle et dialkylazo ayant de 1 à 24 atomes de carbone, ou par les radicaux aryle, arylalkyle, alkylaryle et alkénylaryle ayant de 5 à 24 atomes de carbone, lesdits radicaux pouvant éventuellement être totalement ou partiellement fluorés.

**14.** Composition ionique selon la revendication 1 **caractérisée en ce que** deux substituants R et R' forment ensemble un radical divalent relié à un groupe Y et un groupe Y'.

**15.** Composition ionique selon l'une des revendications 1 ou 12, **caractérisée en ce que** R, R' ou $R^H$ représentent indépendamment les uns des autres un radical alkyle ayant de 1 à 12 atomes de carbone ou un radical alkényle ayant de 2 à 12 atomes de carbone et comprenant éventuellement au moins un hétéroatome O, N ou S dans la chaîne principale ou dans une chaîne latérale, et/ou portant éventuellement un groupe hydroxy, un groupe carbonyle, un groupe amine, un groupe alcoxysilane ou un groupe carboxyle.

**16.** Composition ionique selon l'une des revendications 1 ou 13, **caractérisée en ce que** R et R' forment ensemble un biradical relié à chacune de ses extrémités à un groupe anionique $-SO_2-C^-SO_2X_F$.

**17.** Composition ionique selon l'une des revendications 1 ou 13, **caractérisée en ce qu'**un substituant R, R' ou $R^H$ est un radical polymère.

**18.** Composition selon la revendication 1, **caractérisée en ce qu'**un substituant R ou R' est une unité récurrente d'un polymère.

**19.** Composition selon l'une des revendications 1 ou 12, **caractérisée en ce qu'**un substituant R, R' ou $R^H$ possède au moins un groupement ionophore anionique et/ou au moins un groupement ionophore cationique.

**20.** Composition selon l'une des revendications 1 ou 13, **caractérisée en ce qu'**un substituant R, R' ou $R^H$ comporte au moins une insaturation éthylénique et/ou un groupe condensable et/ou un groupe dissociable par voie thermique, par voie photochimique ou par dissociation ionique.

**21.** Composition selon l'une des revendications 1 ou 12, **caractérisée en ce qu'**un substituant R, R' ou $R^H$ comporte un groupe mésomorphe, un groupe chromophore, un polymère conducteur électronique autodopé ou un alcoxysilane hydrolysable.

**22.** Composition selon l'une des revendications 1 ou 12, **caractérisée en ce qu'**un substituant R, R' ou $R^H$ comporte un groupement susceptible de piéger les radicaux libres, un dipôle dissociant, un couple rédox, un ligand complexant, un groupe optiquement actif, ou un polypeptide optiquement ou biologiquement actif.

**23.** Composition ionique selon la revendication 1 **caractérisée en ce que** Z ou Z' est une unité récurrente d'un polymère.

**24.** Composition ionique selon la revendication 1 **caractérisée en ce que** le solvant de la composition ionique est un solvant liquide aprotique, un polymère polaire ou un de leurs mélanges.

**25.** Composition ionique selon la revendication 24 **caractérisée en ce que** le solvant liquide aprotique est choisi parmi les éthers linéaires et les éthers cycliques, les esters, les nitriles, les dérivés nitrés, les amides, les sulfones, les sulfolanes, les alkylsulfamides et les hydrocarbures partiellement halogénés.

**26.** Composition ionique selon la revendication 24 **caractérisée en ce que** le solvant est un polymère solvatant, réticulé ou non, portant ou non des groupes ioniques greffés.

**27.** Composition ionique selon la revendication 24 **caractérisée en ce que** le solvant est un polymère solvatant choisi parmi

les polyéthers de structure linéaire, peigne ou à blocs, formant ou non un réseau, à base de poly(oxyde d'éthylène),

les copolymères contenant le motif oxyde d'éthylène ou oxyde de propylène ou allylglycidyléther,

les polyphosphazènes,

les réseaux réticulés à base de polyéthylène glycol réticulé par des isocyanates ou les réseaux obtenus par polycondensation et portant des groupements qui permettent l'incorporation de groupements réticulables, et

les copolymères à blocs dans lesquels certains blocs portent des fonctions qui ont des propriétés rédox.

**28.** Composition ionique selon la revendication 24, **caractérisée en ce que** le solvant contient un liquide aprotique et un polymère polaire comprenant des unités contenant au moins un hétéroatome choisi parmi le soufre, l'azote, l'oxygène et le fluor.

**29.** Composition ionique selon la revendication 1 **caractérisée en ce qu'**elle contient un second sel.

**30.** Composition ionique selon la revendication 1 **caractérisée en ce qu'**elle contient une charge minérale ou organique sous forme de poudre ou de fibres.

**31.** Dispositif électrochimique, **caractérisé en ce qu'**il contient comme électrolyte, une composition ionique selon l'une des revendications 1 à 30.

**32.** Générateur électrochimique, comprenant une électrode négative et une électrode positive séparées par un électrolyte, **caractérisé en ce que** l'électrolyte est un composition ionique selon l'une des revendications 1 à 30.

**33.** Générateur selon la revendication 32, **caractérisé en ce que** l'électrode négative est constituée par du lithium métallique, ou par l'un de ses alliages, éventuellement sous forme de dispersion nanométrique dans de l'oxyde de lithium, ou par un nitrure double de lithium et d'un métal de transition, ou par un oxyde à bas potentiel ayant pour formule générale $Li_{1+y+x/3}Ti_{2-x/3}O_4$ ($0 \leq x \leq 1$, $0 \leq y \leq 1$), ou par le carbone et les produits carbonés issus de la pyrolyse de matières organiques.

**34.** Générateur selon la revendication 32, **caractérisé en ce que** la matière active de l'électrode positive est choisie parmi les oxydes de vanadium $VO_x$ ($2 \leq x \leq 2{,}5$), $LiV_3O_8$, $Li_yNi_{1-x}Co_xO_2$, ($0 \leq x \leq 1$ ; $0 \leq y \leq 1$), les spinelles de manganèse $Li_yMn_{1-x}M_xO_2$ (M = Cr, Al, V, Ni, $0 \leq x \leq 0{,}5$ ; $0 \leq y \leq 2$), les polydisulfures organiques, FeS, $FeS_2$, le sulfate de fer $Fe_2(SO_4)_3$, les phosphates et phosphosilicates de fer et de lithium de structure olivine, ou leurs produits de substitution du fer par le manganèse, utilisés seuls ou en mélanges.

**35.** Générateur selon la revendication 32, **caractérisé en ce que** le collecteur de l'électrode positive est en aluminium.

**36.** Générateur selon la revendication 32, **caractérisé en ce que** le composé ionique de l'électrolyte est un sel de métal alcalin.

**37.** Générateur selon la revendication 32, **caractérisé en ce que** les substituants R et R' du composé ionique de l'électrolyte représentent un alkyle, un aryle, un alkylaryle ou un aralkyle ayant de 6 à 20 atomes de carbone.

**38.** Générateur selon la revendication 32, **caractérisé en ce qu'**au moins un substituant R, R' ou $X_F$ du composé ionique de l'électrolyte comprend un groupe mésomorphe ou une insaturation éthylénique et/ou un groupe condensable et/ou un groupe dissociable par voie thermique, par voie photochimique ou par dissociation ionique.

**39.** Générateur selon la revendication 32, **caractérisé en ce que** le sel de l'électrolyte porte au moins un substituant R, R' ou $R^H$ qui est un polymère conducteur électronique autodopé.

**40.** Générateur selon la revendication 32, **caractérisé en ce que** le sel de l'électrolyte porte un substituant R, R' ou $R^H$ qui contient un couple rédox.

**41.** Générateur selon la revendication 32, **caractérisé en ce que** le solvant contient un solvant liquide aprotique polaire.

**42.** Générateur selon la revendication 32, **caractérisé en ce que** le solvant contient un polymère polaire solvatant.

**43.** Générateur selon la revendication 32, **caractérisé en ce que** la cathode est constituée par une composition selon la revendication 1 dans laquelle le composé ionique est un polymère conducteur électronique.

**44.** Supercapacité comprenant un électrolyte et au moins une électrode de carbone à haute surface spécifique, ou une électrode contenant un polymère rédox, **caractérisée en ce que** l'électrolyte est une composition ionique selon la revendication 1.

**45.** Supercapacité selon la revendication 44, **caractérisée en ce que** le cation de l'électrolyte est un ammonium $NH_4^+$, un cation métallique, ou un cation organique comprenant un atome d'azote quaternisé.

**46.** Supercapacité selon la revendication 44, **caractérisée en ce que** l'électrolyte contient un sel dans lequel R, R' et $X_F$ ont un nombre d'atome inférieur à 3, et éventuellement un second sel dans lequel R et/ou R' sont des chaînes alkyles ayant au moins 8 atomes de carbone.

**47.** Supercapacité selon la revendication 44, **caractérisée en ce que** le solvant de l'électrolyte est choisi parmi l'acétonitrile, les carbonates d'alkyle, le mélange carbonate d'éthylène et de diméthoxyéthane.

**48.** Pile à combustible **caractérisée en ce qu'**elle contient comme électrolyte, une composition ionique selon la revendication 1.

**49.** Pile à combustible selon la revendication 48, **caractérisée en ce que** le sel de l'électrolyte a un anion $X_F$-$SO_x$-$C^-$(Z)(Z') ou $X_F$-$SO_x$-$C^-$(Z) (YR) et un cation choisi parmi l'hydronium, l'ammonium, les cations métalliques, et les cations organiques comprenant un atome d'azote quaternisé, (en remplacement partiel de l'hydronium).

**50.** Pile à combustible selon la revendication 49, **caractérisé en ce que** Z et Z' représentent indépendamment l'un de l'autre $-\Phi OC_nF_{2n+1}$, $-\Phi OC_2F_4H$, $-\Phi SC_nF_{2n+1}$, $-\Phi SC_2F_4H$, $-\Phi OCF=CF_2$, $-\Phi SCF=CF_2$, $-C_2F_4H$ ou $CF_2=CF-$, n étant un nombre entier de 1 à 8.

**51.** Dispositif électrophotochimique ayant comme électrolyte, une composition ionique selon la revendication 1.

**52.** Dispositif électrophotochimique selon la revendication 51, **caractérisé en ce que** le sel de la composition ionique est un sel d'un cation choisi dans le groupe constitué par $H^+$, $H_3O^+$, $Na^+$, $K^+$, $NH_4^+$, l'imidazolium, le triazolium, le pyridinium ou le 4-diméthyl aminopyridinium.

**53.** Dispositif électrophotochimique selon la revendication 51, **caractérisé en ce que** le sel de l'électrolyte porte au moins un substituant R, R' ou $R^H$ contenant un couple rédox.

**54.** Dispositif électrophotochimique selon la revendication 51, **caractérisé en ce que** le sel de l'électrolyte a des propriétés de sel fondu.

**55.** Dispositif pour l'affichage optique, **caractérisé en ce qu'**il contient comme électrolyte, une composition selon la revendication 1.

**56.** Dispositif pour l'affichage optique selon la revendication 55, **caractérisé en ce que** le sel de l'électrolyte porte au moins un substituant R, R' ou $R^H$ choisi parmi les radicaux alkyle, aryle, alkylaryle ou arylalkyle ayant de 6 à 20 atomes de carbone.

**57.** Procédé de polymérisation ou de réticulation de monomères ou de prépolymères capables de réagir par voie cationique, **caractérisé en ce que** l'on utilise une composition ionique de l'invention comme photoinitiateur source d'acide catalysant la réaction de polymérisation.

**58.** Procédé selon la revendication 57, **caractérisé en ce que** le cation du sel de la composition ionique est un proton, un oxonium, Li, Mg, Cu, une terre rare, le triméthylsilyle, un ferrocène, un zirconocène, ou un zirconoindocène.

**59.** Procédé selon la revendication 57, **caractérisé en ce que** le cation du sel est un groupement possédant une liaison $\sim N\equiv N^+$ ou -N=N-, un groupement sulfonium, un groupement iodonium, ou un cation arène-ferrocénium

substitué ou non, éventuellement incorporé dans une trame polymérique.

**60.** Procédé selon la revendication 57, **caractérisé en ce qu'**il consiste à mélanger au moins un monomère ou prépolymère capable de polymériser par voie cationique et au moins une composition ionique de l'invention, et à soumettre le mélange obtenu à un rayonnement actinique ou un rayonnement $\beta$.

**61.** Utilisation d'une composition selon la revendication 1 dans laquelle le cation du composé ionique est choisi dans le groupe constitué par H, le lithium, le magnésium, les métaux de transition à l'état divalent ou trivalents, les terres rares, les platinoïdes et leurs couples organo-métalliques, comme catalyseur dans les réactions de Friedel-Crafts, les réactions de Diels-Alder, les réactions d'aldolisation, les additions de Michael, les réactions d'allylation, les réactions de couplage pinacolique, les réactions de glycosylation, les réactions d'ouvertures de cycles des oxétanes, les réactions de métathèse des alcènes, les polymérisations de type Ziegler-Natta, les polymérisations du type métathèse par ouverture de cycle et les polymérisations du type métathèse de diènes acycliques.

**62.** Utilisation d'une composition selon la revendication 1, dans laquelle le cation du sel est choisi dans le groupe constitué par l'imidazolium, le triazolium, le pyridinium et le 4-diméthylamino-pyridinium, ledit cation portant éventuellement un substituant sur les atomes de carbone du cycle, et l'anion du sel porte des substituants qui ont un nombre d'atomes de carbone inférieur ou égal à 4, comme solvant pour des réactions chimiques, photochimiques, électrochimiques, ou photoélectrochimiques.

**63.** Composition de colorant, **caractérisée en ce qu'**elle contient une composition ionique selon la revendication 1 contenant un sel lequel la ou les charges négatives du groupement anionique $X_F$-$SO_x$-$C^-$(Z)(Z'), $X_F$-$SO_x$-$C^-$(YR)(Y'R') ou $X_F$-$SO_x$-$C^-$(Z)(YR) soit sont fixées à une molécule de colorant, soit constituent le contre-ion des charges positives du colorant.

## Patentansprüche

**1.** Ionische Zusammensetzung, umfassend zumindest eine ionische Verbindung in Lösung in einem Lösungsmittel, wobei die Verbindung einen anionischen Anteil umfasst, der zumindest mit einem kationischen Anteil $M^{m+}$ in ausreichendem Ausmaß assoziiert ist, um insgesamt elektronische Neutralität sicher zu stellen, worin die Zusammensetzung **dadurch gekennzeichnet ist, dass** sie über eine Leitfähigkeit verfügt, die bei einer Temperatur zwischen -30 °C und 150 °C über $10^{-5}$ $S.cm^{-1}$ liegt, dass $M^{m+}$ ein Proton, ein Hydronium, ein Hydroxonium, ein Nitrosonium $NO^+$, ein Ammonium $NH_4^+$ oder ein Kation mit einer Wertigkeit m, ausgewählt aus Metallkationen, organischen Kationen und organometallischen Kationen, ist und dass der anionische Anteil der Formel $X_F$-$SO_x$-$C^-$(Z)(Z'), $X_F$-$SO_x$-$C^-$(YR)(Y'R') oder $X_F$-$SO_x C^-$(Z)(YR) entspricht, worin:

- x = 1 oder 2 ist;
- $X_F$ einen einwertigen oder zweiwertigen Rest darstellt, ausgewählt aus der aus unverzweigten, verzweigten oder zyklischen perhalogenierten Resten vom Alkyl-, Alkylaryl-, Oxaalkyl-, Azaalkyl-, Thiaalkyl-, Alkenyl-, Oxaalkenyl-, Azaalkenyl-, Thiaalkenyl- und Dialkylazo-Typ bestehenden Gruppe oder aus der aus organischen Resten bestehenden Gruppe, in denen der Kohlenstoff in $\alpha$-Position zur Gruppe $SO_x$ zumindest ein F-Atom trägt, wobei ein mehrwertiger Rest $X_F$ an mehr als eine Gruppe -$SO_x$Cgebunden ist;
- Z und Z' unabhängig voneinander für einen einwertigen oder mehrwertigen elektronenanziehenden Rest stehen, wobei ein mehrwertiger Rest Z oder Z' an mehrere Gruppen -$C^-SO_x$-$X_F$ gebunden sein kann und der Rest aus:

  * -$NO_2$, -SCN, -$N_3$, -$CF_3$, $R'_FCH_2$- (wobei $R'_F$ ein perfluorierter Rest ist), $CF_2$=CFO-, $CF_2$=CF-S-, $CF_2$-CF-, -$C_2F_4H$, Fluoralkyloxylresten, Perfluoralkyloxylresten, Fluoralkylthioxylresten und Perfluoralkylthioxylresten;
  * Resten, die einen oder mehrere aromatische Kerne umfassen, die gegebenenfalls zumindest ein Stickstoff-, Sauerstoff-, Schwefel- oder Phosphoratom enthalten,

worin die Kerne gegebenenfalls kondensierte Kerne sein können und/oder die Kerne gegebenenfalls zumindest einen Substituenten, ausgewählt aus der aus Halogenen, - $OC_nF_{2n+1}$, -$OC_2F_4H$, -$SC_nF_{2n+1}$, -$SC_2F_4H$, -O-CF=$CF_2$, -SCF=$CF_2$, -CN, -$NO_2$, -SCN, -$N_3$, -$CF_3$, $CF_3CH_2$-, den Aza-, Thia- und Oxa-Resten, Perfluoralkylresten, Fluoralkyloxylresten, Fluoralkylthioxylresten, Alkyl-, Alkenyl-, Oxaalkyl-, Oxaalkenyl-, Azaalkyl-, Azaalkenyl-, Thiaalkyl-, Thiaalkenylresten, Polymerresten und Resten, die zumindest eine kationische Ionophorgruppierung und/oder zu-

mindest eine anionische Ionophorgruppierung bestehenden Gruppe, aufweisen können; ausgewählt ist;

worin ein Substituent Z oder Z' ein einwertiger Rest, ein Teil eines einwertigen Rests, der mit mehreren Gruppierungen $X_F$-$SO_x$-$C^-$ verbunden ist, oder ein Polymersegment sein kann und zwei Substituenten Z oder Z' zusammen eine mehrwertige Gruppierung bilden können, die einen oder mehrere aromatische, gegebenenfalls kondensierte Kerne umfassen, einschließlich oder ausschließlich dem die anionische Ladung tragenden Kohlenstoff;

- Y oder Y' für eine Sulfonyl-, Sulfinyl-, Carbonyl- oder Phosphonylgruppe stehen;
- R ein einwertiger oder mehrwertiger organischer Rest ist und R' = H oder ein einwertiger oder mehrwertiger organischer Rest ist, wobei der organische Rest aus der aus Alkyl-, Alkenyl-, Oxaalkyl-, Oxaalkenyl-, Azaalkyl-, Azaalkenyl-, Thiaalkyl-, Thiaalkenyl- und Dialkylazoresten mit 1 bis 24 Kohlenstoffatomen sowie Aryl-, Arylalkyl-, Alkylaryl- und Alkenylarylresten mit 5 bis 24 Kohlenstoffatomen bestehenden Gruppe ausgewählt ist,

worin jeder der Substituenten R, R', Z oder Z' einen Teil eines Polymers darstellen kann und jeder der Substituenten $X_F$, R, R', Z oder Z' an einen Substituenten $X_F$, R, R', Z oder Z', der von einem anderen anionischen Zentrum getragen wird, gebunden sein kann.

**2.** Ionische Zusammensetzung nach Anspruch 1, **dadurch gekennzeichnet, dass** das Kation der ionischen Verbindung ein Metallkation, ausgewählt aus Kationen von Alkalimetallen, Kationen von Erdalkalimetallen, Kationen von Übergangsmetallen im zweiwertigen oder dreiwertigen Zustand und Kationen von Seltenerden, ist.

**3.** Ionische Zusammensetzung nach Anspruch 1, **dadurch gekennzeichnet, dass** das Kation der ionischen Verbindung ein organisches Kation, ausgewählt aus der aus den Kationen $R''_3O^+$ (Onium), $NR''_4{}^+$ (Ammonium), $R''C(NR''_2)_2{}^+$ (Amidinium), $C(NHR''_2)_3{}^+$ (Guanidinium), $C_5R''_6N^+$ (Pyridinium), $C_3R''_5N_2{}^+$ (Imidazolium), $C_2R''_4N_3{}^+$ (Triazolium), $C_3R''_7N_2{}^+$ (Imidazolinium), $SR''_3{}^+$ (Sulfonium), $PR''_4{}^+$ (Phosphonium), $IR''_2{}^+$ (Iodonium), $(C_6R''_5)_3C^+$ (Carbonium) bestehenden Gruppe ist, worin die Reste R" unabhängig voneinander H oder einen nicht perfluorierten organischen Rest darstellen.

**4.** Ionische Zusammensetzung nach Anspruch 3, **dadurch gekennzeichnet, dass** die Reste R", identisch oder unterschiedlich, aus der aus

- dem Proton, Alkyl-, Alkenyl-, Oxaalkyl-, Oxaalkenyl-, Azaalkyl-, Azaalkenyl-, Thiaalkyl-, Thiaalkenylresten, gegebenenfalls hydrolysierbaren Silaalkylresten, gegebenenfalls hydrolysierbaren Silaalkenylresten und Dialkylazoresten, wobei diese Reste unverzweigt, verzweigt oder zyklisch sein können;
- zyklischen oder heterozyklischen Resten, gegebenenfalls zumindest eine Seitenkette umfassend, die Heteroatome wie Stickstoff, Sauerstoff oder Schwefel umfasst;
- Aryl-, Arylalkyl-, Alkylaryl und Alkenylarylresten, gegebenenfalls Heteroatome im aromatischen Kern oder in einem Substituenten umfassend; und
- Gruppen, die mehrere aromatische oder heterozyklische, kondensierte oder nicht kondensierte Kerne umfassen, die gegebenenfalls zumindest ein Stickstoff-, Sauerstoff-, Schwefel-, oder Phosphoratom enthalten;

bestehenden Gruppe ausgewählt sind.

**5.** Ionische Zusammensetzung nach Anspruch 3, **dadurch gekennzeichnet, dass** das Kation zumindest zwei Reste R" trägt, die nicht H sind, wobei diese Reste gegebenenfalls zusammen einen aromatischen oder nicht aromatischen Ring bilden, der das die kationische Ladung tragende Zentrum inkludiert oder nicht.

**6.** Ionische Zusammensetzung nach Anspruch 3, **dadurch gekennzeichnet, dass** die kationische Gruppe polykationisch ist.

**7.** Ionische Zusammensetzung nach Anspruch 3, **dadurch gekennzeichnet, dass** das Kation ein kationischer heteroaromatischer Ring ist, der zumindest ein quaterniertes Stickstoffatom im Ring aufweist.

**8.** Ionische Zusammensetzung nach Anspruch 7, **dadurch gekennzeichnet, dass** das Kation aus der aus Imidazolium, Triazolium, Pyridinium und 4-Dimethylaminopyridinium bestehenden Gruppe ausgewählt ist und dass sie über diese Kationen gegebenenfalls einen Substituenten an den Kohlenstoffatomen des Rings trägt.

**9.** Ionische Zusammensetzung nach Anspruch 3, **dadurch gekennzeichnet, dass** das Kation eine Bindung -N=N-

oder $\sim N \equiv N^+$, eine Sulfoniumgruppierung, eine Iodoniumgruppierung oder ein substituiertes oder nicht substituiertes Aren-Ferrocen-Kation, gegebenenfalls in eine Polymerkette eingebunden, aufweist.

**10.** Ionische Zusammensetzung nach Anspruch 3, **dadurch gekennzeichnet, dass** das Kation eine Gruppierung 2,2'-[Azobis(2,2'-imidazolinio-2-yl)propan]$^{2+}$ oder 2,2'-Azobis(2-amidiniopropan)$^{2+}$ umfasst.

**11.** Ionische Zusammensetzung nach Anspruch 1, **dadurch gekennzeichnet, dass** $X_F$ ein perhalogenierter Alkylrest mit 1 bis 12 Kohlenstoffatomen oder ein perhalogenierter Alkylarylrest mit 6 bis 9 Kohlenstoffatomen ist, worin diese Reste ein Heteroatom O, N oder S zwischen zwei Kohlenstoffatomen oder am Ende des Rests umfassen können.

**12.** Ionische Zusammensetzung nach Anspruch 1, **dadurch gekennzeichnet, dass** $X_F$ aus der aus $R^HCF_2$-, $R^HCF_2CF_2$-, $R^HCF_2CF(CF_3)$- und $CF_3C(R^H)F$- bestehenden Gruppe ausgewählt ist, wobei $R^H$ einen nicht perhalogenierten organischen Rest darstellt.

**13.** Ionische Zusammensetzung nach einem der Ansprüche 1 oder 12, **dadurch gekennzeichnet, dass** $R^H$ aus der aus Alkyl-, Alkenyl-, Oxaalkyl-, Oxaalkenyl-, Azaalkyl-, Azaalkenyl-, Thiaalkyl-, Thiaalkenyl- und Dialkylazoresten mit 1 bis 24 Kohlenstoffatomen und aus Aryl-, Arylalkyl-, Alkylaryl- und Alkenylarylresten mit 5 bis 24 Kohlenstoffatomen bestehenden Gruppe ausgewählt ist, wobei diese Reste gegebenenfalls zur Gänze oder teilweise fluoriert sein können.

**14.** Ionische Zusammensetzung nach Anspruch 1, **dadurch gekennzeichnet, dass** die zwei Substituenten R und R' zusammen einen zweiwertigen Rest bilden, der an eine Gruppe Y und eine Gruppe Y' gebunden ist.

**15.** Ionische Zusammensetzung nach einem der Ansprüche 1 oder 12, **dadurch gekennzeichnet, dass** R, R' oder $R^H$ unabhängig voneinander einen Alkylrest mit 1 bis 12 Kohlenstoffatomen oder einen Alkenylrest mit 2 bis 12 Kohlenstoffatomen darstellen und gegebenenfalls zumindest ein Heteroatom O, N oder S in der Hauptkette oder in einer Seitenkette umfassen und/oder gegebenenfalls eine Hydroxyl-, Carbonyl-, Amino-, Alkoxysilan- oder Carboxylgruppe aufweisen.

**16.** Ionische Zusammensetzung nach einem der Ansprüche 1 oder 13, **dadurch gekennzeichnet, dass** R und R' zusammen ein Biradikal bilden, das an jedem seiner Enden an eine anionische Gruppe $-SO_2-C^--SO_2X_F$ gebunden ist.

**17.** Ionische Zusammensetzung nach einem der Ansprüche 1 oder 13, **dadurch gekennzeichnet, dass** ein Substituent R, R' oder $R^H$ ein Polymerrest ist.

**18.** Zusammensetzung nach Anspruch 1, **dadurch gekennzeichnet, dass** ein Substituent R oder R' eine Grundeinheit eines Polymers ist.

**19.** Zusammensetzung nach einem der Ansprüche 1 oder 12, **dadurch gekennzeichnet, dass** ein Substituent R, R', oder $R^H$ zumindest eine anionische Ionophorgruppierung und/oder zumindest eine kationische Ionophorgruppierung aufweist.

**20.** Zusammensetzung nach einem der Ansprüche 1 oder 13, **dadurch gekennzeichnet, dass** ein Substituent R, R' oder $R^H$ zumindest eine ethylenische Unsättigung und/oder eine kondensierbare Gruppe und/oder eine durch thermische, photochemische oder ionische Dissoziation dissoziierbare Gruppe umfasst.

**21.** Zusammensetzung nach einem der Ansprüche 1 oder 12, **dadurch gekennzeichnet, dass** ein Substituent R, R' oder $R^H$ eine mesomorphe Gruppe, eine Chromophor-Gruppe, ein selbstdotiertes, elektrisch leitendes Polymer oder ein hydrolysierbares Alkoxysilan umfasst.

**22.** Zusammensetzung nach einem der Ansprüche 1 oder 12, **dadurch gekennzeichnet, dass** ein Substituent R, R' oder $R^H$ eine Gruppierung, die freie Radikale einfangen kann, einen dissoziierenden Dipol, ein Redoxpaar, einen komplexierenden Liganden, eine optisch aktive Gruppe oder ein optisch oder biologisch aktives Polypeptid umfasst.

**23.** Ionische Zusammensetzung nach Anspruch 1, **dadurch gekennzeichnet, dass** Z oder Z' eine Grundeinheit eines

Polymers ist.

**24.** Ionische Zusammensetzung nach Anspruch 1, **dadurch gekennzeichnet, dass** das Lösungsmittel der ionischen Zusammensetzung ein aprotisches, flüssiges Lösungsmittel, ein polares Polymer oder ein Gemisch daraus ist.

**25.** Ionische Zusammensetzung nach Anspruch 24, **dadurch gekennzeichnet, dass** das aprotische, flüssige Lösungsmittel aus unverzweigten Ethern und zyklischen Ethern, Estern, Nitrilen, nitrierten Derivaten, Amiden, Sulfonen, Sulfolanen, Alkylsulfamiden und teilweise halogenierten Kohlenwasserstoffen ausgewählt ist.

**26.** Ionische Zusammensetzung nach Anspruch 24, **dadurch gekennzeichnet, dass** das Lösungsmittel ein solvatisierendes, vernetztes oder nicht vernetztes Polymer ist, das aufpolymerisierte ionische Gruppen trägt oder nicht.

**27.** Ionische Zusammensetzung nach Anspruch 24, **dadurch gekennzeichnet, dass** das Lösungsmittel ein solvatisierendes Polymer ist, ausgewählt aus

Polyethern mit unverzweigter Struktur, Kamm- oder Blockstruktur, die ein Netzwerk bilden oder nicht, basierend auf Polyethylenoxid,

Copolymeren, die ein Ethylenoxid-Motiv oder Propylenoxid-Motiv oder Allylglycidylether-Motiv enthalten,

Polyphosphazenen,

vemetzten Netzwerken auf Basis von auf Isocyanat-vernetztem Polyethylenglykol, oder Netzwerken, die durch Polykondensation erhalten werden und Gruppierungen tragen, die die Einbindung von vernetzbaren Gruppierungen ermöglichen, und

Copolymeren in Blockstruktur, in denen bestimmte Blöcke Funktionen tragen, die Redox-Eigenschaften aufweisen.

**28.** Ionische Zusammensetzung nach Anspruch 24, **dadurch gekennzeichnet, dass** das Lösungsmittel eine aprotische Flüssigkeit und ein polares Polymer enthält, das Einheiten umfasst, die zumindest ein aus Schwefel, Stickstoff, Sauerstoff und Fluor ausgewähltes Heteroatom enthalten.

**29.** Ionische Zusammensetzung nach Anspruch 1, **dadurch gekennzeichnet, dass** sie ein zweites Salz enthält.

**30.** Ionische Zusammensetzung nach Anspruch 1, **dadurch gekennzeichnet, dass** sie einen mineralischen oder organischen Füllstoff in Form von Pulver oder Fasern enthält.

**31.** Elektrochemische Vorrichtung, **dadurch gekennzeichnet, dass** sie als Elektrolyt eine ionische Zusammensetzung nach einem der Ansprüche 1 bis 30 enthält.

**32.** Elektrochemische Zelle, umfassend eine negative Elektrode und eine positive Elektrode, die durch einen Elektrolyt getrennt sind, **dadurch gekennzeichnet, dass** der Elektrolyt eine ionische Zusammensetzung nach einem der Ansprüche 1 bis 30 ist.

**33.** Zelle nach Anspruch 32, **dadurch gekennzeichnet, dass** die negative Elektrode aus metallischem Lithium oder aus einer Legierung davon, gegebenenfalls in Form einer nanometrischen Dispersion in Lithiumoxid, oder aus einem Lithium und Übergangsmetall doppelten Nitrid, oder aus einem Oxid mit niedrigem Potential der allgemeinen Formel $Li_{1+y+x/3}Ti_{2-x/3}O_4$ ($0 \leq x \leq 1$, $0 \leq y \leq 1$) oder aus Kohlenstoff und kohlenstoffhältigen Produkten, die aus der Pyrolyse organischer Materialien hervorgehen, besteht.

**34.** Zelle nach Anspruch 32, **dadurch gekennzeichnet, dass** das aktive Material der positiven Elektrode aus Vanadiumoxiden $VO_x$ ($2 \leq x \leq 2{,}5$), $LiV_3O_8$, $Li_yNi_{1-x}Co_xO_2$ ($0 \leq x \leq 1$; $0 \leq y \leq 1$), Manganspinellen $Li_yMn_{1-x}M_xO_2$ (M = Cr, Al, V, Ni, $0 \leq x \leq 0{,}5$; $0 \leq y \leq 2$), organischen Polydisulfiden, FeS, $FeS_2$, Eisensulfat $Fe_2(SO_4)_3$, Eisenphosphaten und - phosphosilicaten und Lithium mit Olivinstruktur oder Substitutionsprodukten davon, wrin Eisen durch Mangan substituiert ist, alleine oder als Gemisch eingesetzt, ausgewählt ist.

**35.** Zelle nach Anspruch 32, **dadurch gekennzeichnet, dass** der Kollektor der positiven Elektrode aus Aluminium besteht.

**36.** Zelle nach Anspruch 32, **dadurch gekennzeichnet, dass** die ionische Verbindung des Elektrolyts ein Alkalimetallsalz ist.

**37.** Zelle nach Anspruch 32, **dadurch gekennzeichnet, dass** die Substituenten R und R' der ionischen Verbindung des Elektrolyts ein Alkyl, ein Aryl, ein Alkylaryl oder ein Arylalkyl mit 6 bis 20 Kohlenstoffatomen darstellen.

**38.** Zelle nach Anspruch 32, **dadurch gekennzeichnet, dass** zumindest ein Substituent R, R' oder $X_F$ der ionischen Verbindung des Elektrolyts eine mesomorphe Gruppe oder eine ethylenische Unsättigung und/oder eine kondensierbare Gruppe und/oder eine durch thermische, photochemische oder ionische Dissoziation dissoziierbare Gruppe umfasst.

**39.** Zelle nach Anspruch 32, **dadurch gekennzeichnet, dass** das Salz des Elektrolyts zumindest einen Substituenten R, R' oder $R^H$ aufweist, der ein selbstdotiertes elektrisch leitendes Polymer ist.

**40.** Zelle nach Anspruch 32, **dadurch gekennzeichnet, dass** das Salz des Elektrolyts einen Substituenten R, R' oder $R^H$ aufweist, der ein Redoxpaar enthält.

**41.** Zelle nach Anspruch 32, **dadurch gekennzeichnet, dass** das Lösungsmittel ein polares, aprotisches, flüssiges Lösungsmittel enthält.

**42.** Zelle nach Anspruch 32, **dadurch gekennzeichnet, dass** das Lösungsmittel ein solvatisierendes polares Polymer enthält.

**43.** Zelle nach Anspruch 32, **dadurch gekennzeichnet, dass** die Kathode aus einer Zusammensetzung nach Anspruch 1 besteht, worin die ionische Verbindung ein elektrisch leitendes Polymer ist.

**44.** Superkapazität, umfassend einen Elektrolyten und zumindest eine Kohlenstoffelektrode mit hoher spezifischer Oberfläche oder eine Elektrode, die ein Redoxpolymer enthält, **dadurch gekennzeichnet, dass** der Elektrolyt eine ionische Zusammensetzung nach Anspruch 1 ist.

**45.** Superkapazität nach Anspruch 44, **dadurch gekennzeichnet, dass** das Kation des Elektrolyts ein Ammonium $NH_4^+$, ein Metallkation oder ein organisches Kation mit einem quaternierten Stickstoffatom ist.

**46.** Superkapazität nach Anspruch 44, **dadurch gekennzeichnet, dass** der Elektrolyt ein Salz enthält, worin R, R' und $X_F$ eine Atomanzahl unter 3 aufweisen, und gegebenenfalls ein zweites Salz enthält, worin R und/oder R' Alkylketten mit zumindest 8 Kohlenstoffatomen sind.

**47.** Superkapazität nach Anspruch 44, **dadurch gekennzeichnet, dass** das Lösungsmittel des Elektrolyts aus Acetonitril, Alkylcarbonaten oder einem Gemisch aus Ethylencarbonat und Dimethoxyethancarbonat ausgewählt ist.

**48.** Brennstoffzelle, **dadurch gekennzeichnet, dass** sie als Elektrolyt eine ionische Zusammensetzung nach Anspruch 1 enthält.

**49.** Brennstoffzelle nach Anspruch 48, **dadurch gekennzeichnet, dass** das Salz des Elektrolyts ein Anion $X_F$-$SO_x$-$C^-$ (Z) (Z') oder $X_F$-$SO_x$-$C^-$ (Z) (YR) und ein Kation, ausgewählt aus Hydronium, Ammonium, Metallkationen und organischen Kationen, die ein quaterniertes Stickstoffatom umfassen, (als partielle Ersetzung des Hydroniums) aufweist.

**50.** Brennstoffzelle nach Anspruch 49, **dadurch gekennzeichnet, dass** Z und Z' unabhängig voneinander $-\phi OC_nF_{2n+1}$, $-\phi OC_2F_4H$, $-\phi SC_nF_{2n+1}$, $-\phi SC_2F_4H$, $-\phi OCF{=}CF_2$, $-\phi SCF{=}CF_2$, $-C_2F_4H$ oder $CF_2{=}CF-$ darstellen, worin n für eine ganze Zahl von 1 bis 8 steht.

**51.** Elektrochemische Vorrichtung, die eine ionische Zusammensetzung nach Anspruch 1 als Elektrolyt aufweist.

**52.** Elektrochemische Vorrichtung nach Anspruch 51, **dadurch gekennzeichnet, dass** das Salz der ionischen Zusammensetzung ein Salz eines Kations, ausgewählt aus der aus $H^+$, $H_3O^+$, $Na^+$, $K^+$, $NH_4^+$, Imidazolium, Triazolium, Pyridinium und 4-Dimethylaminopyridinium bestehenden Gruppe, ist.

**53.** Elektrochemische Vorrichtung nach Anspruch 51, **dadurch gekennzeichnet, dass** das Salz des Elektrolyts zumindest einen Substituenten R, R' oder $R^H$ aufweist, der ein Redoxpaar enthält.

**54.** Elektrochemische Vorrichtung nach Anspruch 51, **dadurch gekennzeichnet, dass** das Salz des Elektrolyts Eigenschaften von geschmolzenem Salz aufweist.

**55.** Optische Anzeigevorrichtung, **dadurch gekennzeichnet, dass** sie als Elektrolyt eine Zusammensetzung nach Anspruch 1 enthält.

**56.** Optische Anzeigevorrichtung nach Anspruch 55, **dadurch gekennzeichnet, dass** das Salz des Elektrolyts zumindest einen Substituenten R, R' oder $R^H$, ausgewählt aus Alkyl-, Aryl-, Alkylaryl- oder Arylalkylresten mit 6 bis 20 Kohlenstoffatomen, aufweist.

**57.** Verfahren zur Polymerisation oder Vernetzung von Monomeren oder Präpolymeren, die zu einer kationischen Reaktion in der Lage sind, **dadurch gekennzeichnet, dass** eine ionische Zusammensetzung der Erfindung als Photoinitiator-Säurequelle, die die Polymerisationsreaktion katalysiert, verwendet wird.

**58.** Verfahren nach Anspruch 57, **dadurch gekennzeichnet, dass** das Kation des Salzes der ionischen Zusammensetzung ein Proton, ein Oxonium, Li, Mg, Cu, ein Seltenerdelement, Trimethylsilyl, ein Ferrocen, ein Zirconocen oder ein Zirconoindocen ist.

**59.** Verfahren nach Anspruch 57, **dadurch gekennzeichnet, dass** das Kation des Salzes eine Gruppierung, die eine Bindung $\sim N \equiv N^+$ oder -N=N- aufweist, eine Sulfoniumgruppierung, eine lodoniumgruppierung oder ein substituiertes oder nicht substituiertes Aren-Ferrocen-Kation, gegebenenfalls in eine Polymerkette eingebunden, ist.

**60.** Verfahren nach Anspruch 57, **dadurch gekennzeichnet, dass** es darin besteht, zumindest ein Monomer oder Präpolymer, das zu kationischer Polymerisation in der Lage ist, und zumindest eine ionische Zusammensetzung der Erfindung zu vermischen und das erhaltene Gemisch aktinischer Strahlung oder β-Strahlung auszusetzen.

**61.** Verwendung einer Zusammensetzung nach Anspruch 1, in der das Kation der ionischen Verbindung aus der aus H, Lithium, Magnesium, Übergangsmetallen in zweiwertigem oder dreiwertigem Zustand, Seltenerdelementen, Platinmetallen und metallorganischen Paaren davon bestehenden Gruppe als Katalysator bei Friedel-Crafts-Reaktionen, Diels-Alder-Reaktionen, Aldolkondensationsreaktionen, Michael-Additionen, Allylierungs-Reaktionen, Pinakolonbindungs-Reaktionen, Glykosylierungs-Reaktionen, Ringöffnungs-Reaktionen bei Oxetanen, Metathesen-Reaktionen bei Alkenen, Ziegler-Natta-Polymerisationen, Polymerisationen vom Metathesentyp durch Ringöffnung und Polymersiationen vom Metathesentyp von azyklischen Dienen ausgewählt ist.

**62.** Verwendung einer Zusammensetzung nach Anspruch 1, in der das Kation des Salzes aus der aus Imidazolium, Triazolium, Pyridinium und 4-Dimethylaminopyridinium bestehenden Gruppe ausgewählt ist, wobei dieses Kation gegebenenfalls einen Substituenten an den Kohlenstoffatomen des Rings trägt und das Anion des Salzes Substituenten aufweist, die eine Kohlenstoffatomanzahl $\leq 4$ aufweisen, als Lösungsmittel für chemische, photochemische, elektrochemische oder photoelektrochemische Reaktionen.

**63.** Färbemittelzusammensetzung, **dadurch gekennzeichnet, dass** sie eine ionische Zusammensetzung nach Anspruch 1 enthält, die ein Salz enthält, dessen negative(n) Ladung(en) der anionischen Gruppierung $X_F$-$SO_x$-$C^-$(Z)(Z'), $X_F$-$SO_x$-$C^-$(YR)(Y'R') oder $X_F$-$SO_x$-$C^-$ (Z) (YR) entweder an ein Farbmolekül fixiert ist bzw. sind oder das Gegenion zu positiven Ladungen des Färbemittels darstellt bzw. darstellen.

## Claims

**1.** Ionic composition comprising at least one ionic compound in solution in a solvent, the said compound comprising an anionic part associated with at least one cationic part $M^{m+}$ in a number sufficient to ensure the electronic neutrality of the combination, the said composition being **characterized in that** it has a conductivity of greater than $10^{-5}$ $S.cm^{-1}$ at a temperature of between -30°C and 150°C, **in that** $M^{m+}$ is a proton, a hydronium, a hydroxonium, a nitrosonium $NO^+$, an ammonium $-NH_4^+$ or a cation having the valency m chosen from metal cations, organic cations and organometallic cations, and **in that** the anionic part corresponds to one of the formulae $X_F$-$SO_x$-$C^-$(Z)(Z'), $X_F$-$SO_x$-$C^-$(YR)(Y'R') or $X_F$-$SO_x$-$C^-$(Z)(YR) in which :

- x is 1 or 2 ;
- $X_F$ represents a monovalent or multivalent radical chosen from the group consisting of linear, branched or

cyclic perhalogenated radicals of the alkyl, alkylaryl, oxa-alkyl, aza-alkyl, thia-alkyl, alkenyl, oxa-alkenyl, aza-alkenyl, thia-alkenyl or dialkylazo type or from the group consisting of organic radicals in which the carbon α to the $SO_x$ group carries at least one F atom, it being understood that a multivalent $X_F$ radical is connected to more than one $-SO_xC-$ group ;

- Z and Z' represent, independently of one another, a monovalent or multivalent electron-withdrawing radical, it being understood that a multivalent Z or Z' radical can be connected to several $-C^-SO_x-X_F$ groups ; said radical being chosen from :

  • $-NO_2$, -SCN, $-N_3$, $-CF_3$, $R'_FCH_2-$ ($R'_F$ being a perfluorinated radical), $CF_2=CFO-$, $CF_2=CF-S-$, $CF_2=CF-$, $-C_2F_4H$, fluoroalkyloxy radicals, perfluoroalkyloxy radicals, fluoroalkylthioxy radicals and perfluoroalkylthioxy radicals ;
  • radicals comprising one or more aromatic nuclei optionally comprising at least one nitrogen, oxygen, sulfur or phosphorus atom, it being possible for the said nuclei optionally to be condensed nuclei and/or it being possible for the said nuclei optionally to carry at least one substituent chosen from the group consisting of halogens, $-OC_nF_{2n+1}$, $-OC_2F_4H$, $-SC_nF_{2n+1}$, $-SC_2F_4H$, $-O-CF=CF_2$, $-SCF=CF_2$, -CN, $-NO_2$, -SCN, $-N_3$, $-CF_3$, $CF_3CH_2-$, aza, thia and oxa radicals, perfluoroalkyl radicals, fluoroalkyloxy radicals, fluoroalkylthioxy radicals, alkyl, alkenyl, oxa-alkyl, oxa-alkenyl, aza-alkyl, aza-alkenyl, thia-alkyl or thia-alkenyl radicals, polymer radicals, and radicals possessing at least one cationic ionophoric group and/or at least one anionic ionophoric group ;

it being understood that a Z or Z' substituent can be a monovalent radical, a portion of a multivalent radical connected to several $X_F-SO_x-C^-$< groups, or a segment of a polymer, and that two Z and Z' substituents can together form a multivalent group comprising a nucleus or several optionally condensed aromatic nuclei, including or not including the carbon carrying the anionic charge ;

- Y or Y' represent a sulfonyl, sulfinyl, carbonyl or phosphonyl group ;
- R is a monovalent or multivalent organic radical and R' is H or a monovalent or multivalent organic radical, said organic radical beeing chosen from the group consisting of alkyl, alkenyl, oxa-alkyl, oxa-alkenyl, aza-alkyl, aza-alkenyl, thia-alkyl, thia-alkenyl and dialkylazo radicals having from 1 to 24 carbon atoms or of aryl, arylalkyl, alkylaryl and alkenylaryl radicals having from 5 to 24 carbon atoms,

it being understood that each of the substituents R, R', Z or Z' can form part of a polymer and that each of the substituents $X_F$, R, R', Z or Z' can be connected to a substituent $X_F$, R, R', Z or Z' carried by another anionic center.

**2.** Ionic composition according to Claim 1, **characterized in that** the cation of the ionic compound is a metal cation chosen from alkali metal cations, alkaline earth metal cations, transition metal cations in the divalent or trivalent state, and rare earth cations.

**3.** Ionic composition according to Claim 1, **characterized in that** the cation of the ionic compound is an organic cation chosen from the group consisting of the $R''_3O^+$ (onium), $NR''_4{}^+$ (ammonium), $R''C(NHR''_2)_2{}^+$ (amidinium), $C(NHR''_2)_3{}^+$ (guanidinium), $C_5R''_6N^+$ (pyridinium), $C_3R''_5N_2{}^+$ (imidazolium), $C_2R''_4N_3{}^+$ (triazolium), $C_3R''_7N_2{}^+$ (imidazolinium), $SR''_3{}^+$ (sulfonium), $PR''_4{}^+$ (phosphonium), $IR''_2{}^+$ (iodonium) or $(C_6R''_5)_3C^+$ (carbonium) cations, the R" radicals independently representing H or a nonperfluorinated organic radical.

**4.** Ionic composition according to Claim 3, **characterized in that** the R" radicals, which are identical or different, are chosen from the group consisting of :

- the proton, alkyl, alkenyl, oxa-alkyl, oxa-alkenyl, aza-alkyl, aza-alkenyl, thia-alkyl, thia-alkenyl, optionally hydrolyzable sila-alkyl or optionally hydrolyzable sila-alkenyl radicals and dialkylazo radicals, it being possible for the said radicals to be linear, branched or cyclic ;
- cyclic or heterocyclic radicals optionally comprising at least one side chain comprising heteroatoms, such as nitrogen, oxygen or sulfur ;
- aryl, arylalkyl, alkylaryl and alkenylaryl radicals, optionally comprising heteroatoms in the aromatic nucleus or in a substituent ;
- groups comprising several condensed or noncondensed, aromatic or heterocyclic nuclei, optionally comprising at least one nitrogen, oxygen, sulfur or phosphorus atom.

**5.** Ionic composition according to Claim 3, **characterized in that** the cation carries at least two R" radicals other than

H, these radicals optionally together forming an aromatic or nonaromatic ring encompassing or not encompassing the center carrying the cationic charge.

**6.** Ionic composition according to Claim 3, **characterized in that** the cationic group is polycationic.

**7.** Ionic composition according to Claim 4, **characterized in that** the cation is a cationic heterocycle of aromatic nature comprising at least one quaternized nitrogen atom in the ring.

**8.** Ionic composition according to Claim 7, **characterized in that** the cation is chosen from the group consisting of imidazolium, triazolium, pyridinium and 4-(dimethylamino)pyridinium and of the said cations optionally carrying a substituent on the carbon atoms of the ring.

**9.** Ionic composition according to Claim 3, **characterized in that** the cation possesses an -N=N- or $\sim N\equiv N^+$ linkage, a sulfonium group, an iodonium group or an areneferrocenium cation which is substituted or unsubstituted, optionally incorporated in a polymeric backbone.

**10.** Ionic composition according to Claim 3, **characterized in that** the cation comprises a 2,2'[Azobis(2-2'-imidazolinio-2-yl)propane]$^{2+}$ or 2,2'-Azobis(2-amidiniopropane)$^{2+}$ group.

**11.** Ionic composition according to Claim 1, **characterized in that** $X_F$ is a perhalogenated alkyl radical having from 1 to 12 carbon atoms or a perhalogenated alkylaryl radical having from 6 to 9 carbon atoms, it being possible for the said radicals to comprise an O, N or S heteroatom between two carbon atoms or at the end of the radical.

**12.** Ionic composition according to Claim 1, **characterized in that** $X_F$ is chosen from the group consisting of $R^HCF_2$-, $R^HCF_2CF_2$-, $R^HCF_2CF(CF_3)$- and $CF_3C(R^H)F$-, $R^H$ representing a nonperhalogenated organic radical.

**13.** Ionic composition according to Claim 1 or 12, **characterized in that** $R^H$ said is chosen from the group consisting of alkyl, alkenyl, oxa-alkyl, oxa-alkenyl, aza-alkyl, aza-alkenyl, thia-alkyl, thia-alkenyl and dialkylazo radicals having from 1 to 24 carbon atoms, or of aryl, arylalkyl, alkylaryl and alkenylaryl radicals having from 5 to 24 carbon atoms, said radicals beeing optionally completely or partly fluorinated.

**14.** Ionic composition according to Claim 1, **characterized in that** two substituents R and R' form together a divalent radical linked to a Y group or a Y' group.

**15.** Ionic composition according to Claim 1 or 12, **characterized in that** R, R' or $R^H$ represent, independently of one another, an alkyl radical having from 1 to 12 carbon atoms or an alkenyl radical having from 2 to 12 carbon atoms and optionally comprising at least one 0, N or S heteroatom in the main chain or in a side chain and/or optionally carrying a hydroxyl group, a carbonyl group, an amine group, an alkoxysilane group or a carboxyl group.

**16.** Ionic composition according to Claim 1 or 13, **characterized in that** R and R' together form a biradical connected, at each of its ends, to an $-SO_2-C^-SO_2X_F$ anionic group.

**17.** Ionic composition according to Claim 1 or 13, **characterized in that** an R, R' or $R^H$ substituent is a polymer radical.

**18.** Composition according to Claim 1, **characterized in that** an R or R' substituent is a repeat unit of a polymer.

**19.** Composition according to Claim 1 or 12, **characterized in that** an R, R' or $R^H$ substituent possesses at least one anionic ionophoric group and/or at least one cationic ionophoric group.

**20.** Composition according to Claim 1 or 13, **characterized in that** an R, R' or $R^H$ substituent comprises at least one ethylenic unsaturated and/or one condensable group and/or one group dissociable by the thermal route, by the photochemical route or by ionic dissociation.

**21.** Composition according to Claim 1 or 12, **characterized in that** an R, R' or $R^H$ substituent comprises a mesomorphic group, a chromophoric group, a self-doped electronic conducting polymer or a hydrolyzable alkoxysilane.

**22.** Composition according to Claim 1 or 12, **characterized in that** an R, R' or $R^H$ substituent comprises a group capable of scavenging free radicals, a dissociating dipole, a redox couple, a complexing ligand, an optically active

group, or an optically or biologically active polypeptide.

**23.** Ionic composition according to Claim 1, **characterized in that** Z or Z' is a repeat unit of a polymer.

**24.** Ionic composition according to Claim 1, **characterized in that** the solvent of the ionic composition is an aprotic liquid solvent, a polar polymer or one of their mixtures.

**25.** Ionic composition according to Claim 24, **characterized in that** the aprotic liquid solvent is chosen from linear ethers and cyclic ethers, esters, nitriles, nitro derivatives, amides, sulfones, sulfolanes, alkylsulfamides and partially halogenated hydrocarbons.

**26.** Ionic composition according to Claim 24, **characterized in that** the solvent is a crosslinked or noncrosslinked solvating polymer carrying or not carrying grafted ionic groups.

**27.** Ionic composition according to Claim 24, **characterized in that** the solvent is a solvating polymer chosen from

- polyethers with a linear, comb or block structure, forming or not forming a network, based on poly(ethylene oxide),
- copolymers comprising the ethylene oxide or propylene oxide or allyl glycidyl ether unit,
- polyphosphazenes,
- crosslinked networks based on poly(ethylene glycol) crosslinked by isocyanates or networks obtained by polycondensation and carrying groups which make possible the incorporation of crosslinkable groups, and
- block copolymers in which some blocks carry functional groups which have redox properties.

**28.** Ionic composition according to Claim 24, **characterized in that** the solvent comprises an aprotic liquid and a polar polymer comprising units comprising at least one heteroatom chosen from sulfur, nitrogen, oxygen and fluorine.

**29.** Ionic composition according to Claim 1, **characterized in that** it comprises a second salt.

**30.** Ionic composition according to Claim 1, **characterized in that** it comprises an inorganic or organic filler in the form of a powder or of fibers.

**31.** Electrochemical device, **characterized in that** it comprises, as electrolyte, an ionic composition according to any of Claims 1 to 30.

**32.** Electrochemical generator comprising a negative electrode and a positive electrode separated by an electrolyte, **characterized in that** the electrolyte is an ionic composition according to one of Claims 1 to 30.

**33.** Generator according to Claim 32, **characterized in that** the negative electrode is composed of metallic lithium or of one of its alloys, optionally in the form of a nanometric dispersion in lithium oxide, or of a double lithium and transition metal nitride, or of an oxide of low potential having the general formula $Li_{1+y+x/3}Ti_{2-x/3}O_4$ ($0 \leq x \leq 1$, $0 \leq y \leq 1$), or of carbon and carbonaceous products resulting from the pyrolysis of organic matter.

**34.** Generator according to Claim 32, **characterized in that** the active material of the positive electrode is chosen from vanadium oxides $VO_x$ ($2 \leq x \leq 2.5$), $LiV_3O_8$, $Li_yNi_{1-x}Co_xO_2$, ($0 \leq x \leq 1$ ; $0 \leq y \leq 1$), manganese spinels $Li_yMn_{1-x}M_xO_2$ (M = Cr, Al, V, Ni, $0 \leq x \leq 0.5$ ; $0 \leq y \leq 2$), organic polydisulfides, FeS, $FeS_2$, iron sulfate $Fe_2(SO_4)_3$, iron and lithium phosphates and phosphosilicates of olivine structure, or their products of substitution of the iron by manganese, used alone or as mixtures.

**35.** Generator according to Claim 32, **characterized in that** the collector of the positive electrode is made of aluminum.

**36.** Generator according to Claim 32, **characterized in that** the ionic compound of the electrolyte is an alkali metal salt.

**37.** Generator according to Claim 32, **characterized in that** the R and R' substituents of the ionic compound of the electrolyte represent an alkyl, an aryl, an alkylaryl or an aralkyl having from 6 to 20 carbon atoms.

**38.** Generator according to Claim 32, **characterized in that** at least one R, R' or $X_F$ substituent of the ionic compound of the electrolyte comprises a mesomorphic group or an ethylenic unsaturation and/or a condensable group and/or a group dissociable by the thermal route, by the photochemical route or by ionic dissociation.

**39.** Generator according to Claim 32, **characterized in that** the salt of the electrolyte carries at least one R, R' or $R^H$ substituent which is a self-doped electronic conducting polymer.

**40.** Generator according to Claim 32, **characterized in that** the salt of the electrolyte carries an R, R' or $R^H$ substituent which comprises a redox couple.

**41.** Generator according to Claim 32, **characterized in that** the solvent comprises a polar aprotic liquid solvent.

**42.** Generator according to Claim 32, **characterized in that** the solvent comprises a solvating polar polymer.

**43.** Generator according to Claim 32 **characterized in that** the cathode is composed of a composition according to Claim 1 in which the ionic compound is an electronic conducting polymer.

**44.** Supercapacitor comprising an electrolyte and at least one carbon electrode with a high specific surface or one electrode comprising a redox polymer, **characterized in that** the electrolyte is an ionic composition according to Claim 1.

**45.** Supercapacitor according to Claim 44, **characterized in that** the cation of the electrolyte is an ammonium $NH_4^+$, a metal cation or an organic cation comprising a quaternized nitrogen atom.

**46.** Supercapacitor according to Claim 44, **characterized in that** the electrolyte comprises a salt in which R, R' and $X_F$ have an atom number of less than 3 and optionally a second salt in which R and/or R' are alkyl chains having at least 8 carbon atoms.

**47.** Supercapacitor according to Claim 44, **characterized in that** the solvent of the electrolyte is chosen from acetonitrile, alkyl carbonates or the ethylene carbonate and dimethoxyethane mixture.

**48.** Fuel cell, **characterized in that** it comprises, as electrolyte, an ionic composition according to Claim 1.

**49.** Fuel cell according to Claim 48, **characterized in that** the salt of the electrolyte has an $X_F$-$SO_x$-$C^-$-(Z)(Z') or $X_F$-$SO_x$-$C^-$(Z)(YR) anion and a cation chosen from hydronium, ammonium, metal cations and organic cations comprising a quaternized nitrogen atom (as partial replacement for hydronium).

**50.** Fuel cell according to Claim 49, **characterized in that** Z and Z' represent, independently of one another, -$\Phi OC_nF_{2n+1}$, -$\Phi OC_2F_4H$, -$\Phi SC_nF_{2n+1}$, -$\Phi SC_2F_4H$, -$\Phi OCF=CF_2$, -$\Phi SCF=CF_2$, -$C_2F_4H$, or $CF_2=CF$-, n being an integer from 1 to 8.

**51.** Electrophotochemical device having, as electrolyte, an ionic composition according to Claim 1.

**52.** Electrophotochemical device according to Claim 51, **characterized in that** the salt of the ionic composition is a salt of a cation chosen from the group consisting of $H^+$, $H_3O^+$, $Na^+$, $K^+$, $NH_4^+$, imidazolium, triazolium, pyridinium or 4-(dimethylamino)pyridinium.

**53.** Electrophotochemical device according to Claim 51, **characterized in that** the salt of the electrolyte carries at least one R, R' or $R^H$ substituent comprising a redox couple.

**54.** Electrophotochemical device according to Claim 51, **characterized in that** the salt of the electrolyte has molten salt properties.

**55.** Device for optical display, **characterized in that** it comprises, as electrolyte, a composition according to Claim 1.

**56.** Device for optical display according to Claim 55, **characterized in that** the salt of the electrolyte carries at least one R, R' or $R^H$ substituent chosen from alkyl, aryl, alkylaryl or arylalkyl radicals having from 6 to 20 carbon atoms.

**57.** Process for the polymerization or for the crosslinking of monomers or of prepolymers capable of reacting by the cationic route, **characterized in that** use is made of an ionic composition of the invention as photoinitiator source

of acid catalyzing the polymerization reaction.

**58.** Process according to Claim 57, **characterized in that** the cation of the salt of the ionic composition is a proton, an oxonium, Li, Mg, Cu, a rare earth [lacuna], trimethylsilyl, a ferrocene, a zirconocene or a zirconoindocene.

**59.** Process according to Claim 57, **characterized in that** the cation of the salt is a group possessing an $\sim N{\equiv}N^+$ or - N=N- linkage, a sulfonium group, an iodonium group or an areneferrocenium cation which is substituted or unsubstituted, optionally incorporated in a polymeric backbone.

**60.** Process according to Claim 57, **characterized in that** it consists in mixing at least one monomer or prepolymer capable of polymerizing by the cationic route and at least one ionic composition of the invention and in subjecting the mixture obtained to actinic radiation or β radiation.

**61.** Use of a composition according to Claim 1, in which the cation of the ionic compound is chosen from the group consisting of H, lithium, magnesium, transition metals in the divalent or trivalent state, rare earths, platinoids and their organometallic couples, as catalyst in Friedel-Crafts reactions, Diels-Alder reactions, aldol reactions, Michael additions, allylation reactions, pinacol coupling reactions, glycosylation reactions, ring opening reactions of oxetanes, metathesis reactions of alkenes, polymerizations of Ziegler-Natta type, polymerizations of the metathesis type by ring opening and polymerizations of the metathesis type of acyclic dienes.

**62.** Use of a composition according to Claim 1, in which the cation of the salt is chosen from the group consisting of imidazolium, triazolium, pyridinium and 4-(dimethylamino)pyridinium, the said cation optionally carrying a substituent on the carbon atoms of the ring, and the anion of the salt carries substituents which have a number of carbon atoms of less than or equal to 4, as solvent for chemical, photochemical, electrochemical or photoelectrochemical reactions.

**63.** Dye composition, **characterized in that** it comprises an ionic composition according to Claim 1 comprising a salt wherein the negative charge or charges of the $X_F$-$SO_x$-$C^-$ (Z)(Z'), $X_F$-$SO_x$-$C^-$(YR)(Y'R') or $X_F$-$SO_x$-$C^-$(Z)(YR) anionic group are either attached to a dye molecule or constitute the counterion of the positive charges of the dye.